(19) Europäisches Patentamt
European Patent Office
Office européen des brevets

(11) **EP 4 258 002 B1**

(12) **EUROPEAN PATENT SPECIFICATION**

(45) Date of publication and mention
of the grant of the patent:
**30.10.2024   Bulletin 2024/44**

(21) Application number: **22305465.1**

(22) Date of filing: **06.04.2022**

(51) International Patent Classification (IPC):
**G01R 33/48** $^{(2006.01)}$     **A61B 5/0536** $^{(2021.01)}$
**G01N 3/08** $^{(2006.01)}$      **A61B 5/055** $^{(2006.01)}$
**A61B 5/0535** $^{(2021.01)}$    **A61B 5/00** $^{(2006.01)}$
**G01R 33/56** $^{(2006.01)}$

(52) Cooperative Patent Classification (CPC):
**G01R 33/4808; A61B 5/0535; A61B 5/055;
A61B 5/7264; G01R 33/5608**

(54) **ESTIMATING A DISTRIBUTION OF CONDUCTIVITY OVER A PORTION OF A TISSUE OF A BODY**

SCHÄTZUNG DER LEITFÄHIGKEITSVERTEILUNG ÜBER EINEN TEIL EINES GEWEBES EINES KÖRPERS

ESTIMATION D'UNE DISTRIBUTION DE LA CONDUCTIVITÉ SUR UNE PARTIE D'UN TISSU D'UN CORPS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**11.10.2023   Bulletin 2023/41**

(73) Proprietors:
• **Université de Lorraine**
  **54000 Nancy (FR)**
• **INSTITUT NATIONAL DE LA SANTE ET DE LA
  RECHERCHE
  MEDICALE**
  **75654 Paris Cedex 13 (FR)**
• **Centre Hospitalier Régional Universitaire de
  Nancy**
  **54000 Nancy (FR)**

(72) Inventors:
• **ROUSSEL, Benjamin**
  **NANCY (FR)**
• **OSTER, Julien**
  **VILLERS LES NANCY (FR)**
• **FELBLINGER, Jacques**
  **MEREVILLE (FR)**

(74) Representative: **Bandpay & Greuter**
  **11 rue Christophe Colomb
  75008 Paris (FR)**

(56) References cited:
**WO-A2-2007/017779     US-A1- 2018 310 854**

**US-B2- 8 948 875**

• **IBRAGIMOV Z ET AL: "Reconstruction of the
  Conductivity Profile of the Human Head by a
  Non-Invasive Method", 2019 INTERNATIONAL
  CONFERENCE ON ELECTROMAGNETICS IN
  ADVANCED APPLICATIONS (ICEAA), IEEE, 9
  September 2019 (2019-09-09), pages 881,
  XP033639202, DOI: 10.1109/ICEAA.2019.8879241**
• **KISTENEV YU V ET AL: "Investigation of the
  electric field distribution in the human brain
  based on MRI and EEG data", PROCEEDINGS OF
  SPIE; [PROCEEDINGS OF SPIE ISSN 0277-786X
  VOLUME 10524], SPIE, US, vol. 10614, 16 April
  2018 (2018-04-16), pages 106141P - 106141P,
  XP060103953, ISBN: 978-1-5106-1533-5, DOI:
  10.1117/12.2303464**
• **YUQING WAN ET AL: "A feasibility study of
  magnetic resonance driven electrical impedance
  tomography using a phantom", PHYSIOLOGICAL
  MEASUREMENT, INSTITUTE OF PHYSICS
  PUBLISHING, BRISTOL, GB, vol. 34, no. 6, 29 May
  2013 (2013-05-29), pages 623 - 644, XP020245478,
  ISSN: 0967-3334, DOI:
  10.1088/0967-3334/34/6/623**
• **JIAEN LIU ET AL: "Electrical Properties
  Tomography Based on B1 Maps in MRI:
  Principles, Applications, and Challenges", IEEE
  TRANSACTIONS ON BIOMEDICAL
  ENGINEERING, vol. 64, no. 11, 21 August 2017
  (2017-08-21), USA, pages 2515 - 2530,
  XP055690456, ISSN: 0018-9294, DOI:
  10.1109/TBME.2017.2725140**

EP 4 258 002 B1

- **LI XIUYAN ET AL: "An image reconstruction framework based on deep neural network for electrical impedance tomography", 2017 IEEE INTERNATIONAL CONFERENCE ON IMAGE PROCESSING (ICIP), IEEE, 17 September 2017 (2017-09-17), pages 3585 - 3589, XP033323243, DOI: 10.1109/ICIP.2017.8296950**

## Description

### TECHNICAL FIELD

The disclosure relates to the field of computer programs and systems, and more specifically to a method, system and program for estimating a distribution of conductivity over a portion of a tissue of a body.

### BACKGROUND

The development of reliable and non-invasive methods for the estimation of electrical properties of inner tissues has attracted more and more attention due to its suitability for several clinical applications including, e.g., cardiac applications such as Electrocardiographic Imaging (ECGI), stroke type differentiation and detection of cancerous cells, among others. The concept of non-invasive method is well known in the art.

Some of the existing state of the art include Electrical Property Tomography (EPT) techniques and Electrical Impedance Tomography (EIT) techniques.

EPT techniques include, e.g., MRI-based techniques, also known as "MR-EPT" in some applications. MRI stands for "Magnetic Resonance Image" or "Magnetic Resonance Imaging". However, the performance for imaging electrical properties with EPT techniques is highly dependent of the frequency of the stimulation to the body. For instance at low frequencies, the tissues are mainly conductors and hence lack characterization.

Electrical Impedance Tomography (EIT) techniques aim at imaging biological tissues according to their electrical properties. In EIT, an electrical current is produced inside the body (either directly injected or induced by varying magnetic fields -IC-EIT-) and the developing potentials are measured at the surface. The estimation of the body electrical property map is performed by solving an inverse problem, which is ill-posed. The physical laws governing the phenomenon linking the varying magnetic fields, in the case of IC-EIT, and the developing potentials measured at the surface involve Partial Differential Equations (PDEs) and its solution may rely on using Finite Element Methods (FEM).

Also known in the art are:

- Ibragimov Z., "Reconstruction of the Conductivity Profile of the Human Head by a Non-Invasive Method", 2019 International Conference on Electromagnetics in Advanced Applications, IEEE, 09.09.2019, discloses a method for the estimation of the conductivity profile;
- Patent US 2018/310854 A1 which discloses a method of estimating potential distribution over a cortical surface of a brain of a subject;
- Kistenev Yu. V. et. al., "Investigation of the electric field distribution in the human brain based on MRI and EEG data", Proceedings of SPIE, vol. 10614, 16.04.2018, which discloses an approach to restoration of the spatial-temporal distribution of electric field in the human brain;
- Yuqing Wan et. al., "A feasibility study of magnetic resonance driven electrical impedance tomography using a phantom", Physiological Measurement, vol. 34, no. 6, 29.05.2013, which discloses a method of measuring the electrical properties using a magnetic resonance (MR) scanner without current injection;
- WO 2007/017779 A2 which discloses an electric impedance imaging system in which the electrical permittivity and/or conductivity distribution is derived on the basis of magnetic resonance signals;
- Liu Jiaen, "Electrical Properties Tomography Based on B1 Maps in MRI: Principles, Applications and Challenges", IEEE Transactions on Biomedical Engineering, vol. 64, no. 11, 21.08.2017, discloses a review of the electrical properties tomography (EPT) technique; and
- Patent US 8948875 B2 discloses a method for non-invasive, electrical deep-brain stimulation.

Within this context, there is still a need for an improved method for estimating a distribution of conductivity over a portion of a tissue of a body.

### SUMMARY

The invention is set out in the appended claims.

### BRIEF DESCRIPTION OF THE DRAWINGS

Non-limiting examples will now be described in reference to the accompanying drawings, where:

- FIG. 1 shows a flowchart of an example of the method;
- FIG. 2 shows an example of the system; and

- FIG.s 3 to 10 illustrate the method.

## DETAILED DESCRIPTION

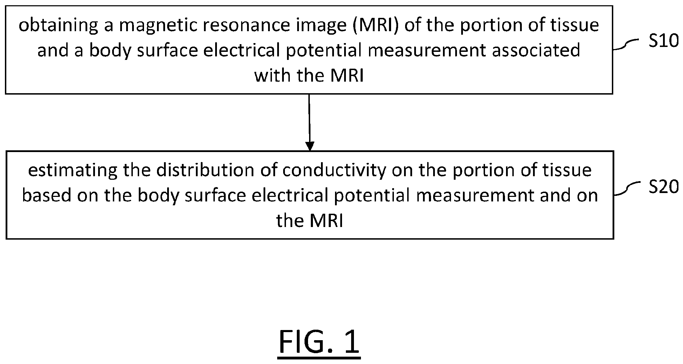

With reference to the flowchart of FIG. 1, it is proposed a computer-implemented method for estimating a distribution of conductivity over a portion of a tissue of a body. The method comprises obtaining S10 a magnetic resonance image (MRI) of the portion of tissue and a body surface electrical potential measurement associated with the MRI. The method also comprises estimating S20 the distribution of conductivity on the portion of tissue based on the body surface electrical potential measurement and on the MRI.

Such method improves the estimation of the distribution of conductivity on the portion. Indeed, the method takes as input the obtained MRI of a portion of a tissue of a body (*e.g.* of a human, *e.g.* a human patient) and the obtained measurement, which measures an electrical surface potential at the surface of the body portion. The method thus has as input data about the morphology and physiology of the body (i.e. captured by the MRI) and data about the electrical behavior of the body when subject to the electrical-magnetic field created by the magnetic resonance imaging from which the obtained MRI stems. Such data is however partial, since obtained at the surface of the body, and thus does not necessarily capture the electrical behavior of the body portion completely (e.g. does not necessarily capture the electrical behavior inside the body portion). However, the method uses these two inputs, that is the MRI and the measurement, to combine these morphological and surface electrical data to obtain the conductivity distribution estimated at S20. This distribution is a distribution of conductivity on the portion of tissue, i.e. captures the electrical behavior of the whole body portion, not a surface behavior, when subject to the MRI electro-magnetic field.

The method thus provides a reliable estimation while being non-invasive to a patient. Indeed, on one hand, the method incorporates the morphology of the portion of the tissue (captured by the MRI image) and associates it with the surface measurements, to provide a more complete and accurate body conductivity estimation that respects said morphology. This improves accuracy. On the other hand, as the method is based on the body surface electrical potential measurement and on an MRI, the method does not rely on directly applying a current to the body, thus being non-invasive and avoiding current-based artifacts. Indeed, MRI and surface electrical measurement (e.g. by means of surface electrodes), are known to be non-invasive methods. The method thus forms an improved, non-invasive, method for processing an MRI of a tissue portion and associated body surface electrical potential measurement to obtain a distribution of conductivity values over the portion of tissue represented by the MRI.

The method may be used in method for measuring the physical conductivity of the body that comprises the method. The method for measuring may comprise acquiring the MRI by applying any MRI imaging process known in the art, and the body surface electrical potential measurement associated with the MRI. The method for measuring may comprise placing surface electrodes for obtaining the measurement. The surface electrodes may be placed in any manner so as to obtain a body surface electrical potential measurement from the magnetic field applied by the MRI imaging. The method for measuring may then comprise using the method for estimating the distribution of conductivity over a portion of a tissue of a body. The method for measuring may then further comprise changing the placement of the surface electrodes according to an uncertainty of the estimated distribution of conductivity, as further discussed hereinafter. The use of the method for estimating the distribution of conductivity and the changing of placement of the surface electrodes may be iterated, as further discussed hereinafter.

The method is computer-implemented. This means that steps (or substantially all the steps) of the method are executed by at least one computer, or any system alike. Thus, steps of the method are performed by the computer, possibly fully automatically, or, semi-automatically. In examples, the triggering of at least some of the steps of the method may be performed through user-computer interaction. The level of user-computer interaction required may depend on the level of automatism foreseen and put in balance with the need to implement user's wishes. In examples, this level may be user-defined and/or pre-defined.

Atypical example of computer-implementation of the method is to perform the method with a system adapted for this purpose. The system may comprise a processor coupled to a memory and a graphical user interface (GUI), the memory having recorded thereon a computer program comprising instructions for performing the method. The memory may also store a database. The memory is any hardware adapted for such storage, possibly comprising several physical distinct parts (e.g. one for the program, and possibly one for the database).

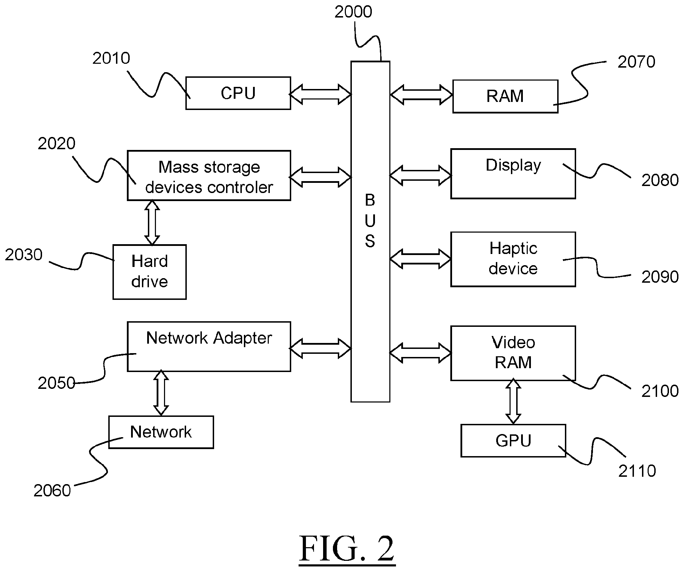

FIG. 2 shows an example of the system, wherein the system is a client computer system, e.g. a workstation of a user.

The client computer of the example comprises a central processing unit (CPU) 2010 connected to an internal communication BUS 2000, a random access memory (RAM) 2070 also connected to the BUS. The client computer is further provided with a graphical processing unit (GPU) 2110 which is associated with a video random access memory 2100 connected to the BUS. Video RAM 2100 is also known in the art as frame buffer. A mass storage device controller 2020 manages accesses to a mass memory device, such as hard drive 2030. Mass memory devices suitable for tangibly embodying computer program instructions and data include all forms of nonvolatile memory, including by way of example semiconductor memory devices, such as EPROM, EEPROM, and flash memory devices; magnetic disks such as internal hard disks and removable disks; magneto-optical disks. Any of the foregoing may be supplemented by, or incorporated in, specially designed ASICs (application-specific integrated circuits). A network adapter 2050 manages accesses to a network 2060. The client computer may also include a haptic device 2090 such as cursor control device, a keyboard or the like. A cursor control device is used in the client computer to permit the user to selectively position a cursor at any desired location on display 1080. In addition, the cursor control device allows the user to select various commands, and input control signals. The cursor control device includes a number of signal generation devices for input control signals to system. Typically, a cursor control device may be a mouse, the button of the mouse being used to generate the signals. Alternatively or additionally, the client computer system may comprise a sensitive pad, and/or a sensitive screen.

The computer program may comprise instructions executable by a computer, the instructions comprising means for causing the above system to perform the method. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the method by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the method. The computer program may alternatively be stored and executed on a server of a cloud computing environment, the server being in communication across a network with one or more clients. In such a case a processing unit executes the instructions comprised by the program, thereby causing the method to be performed on the cloud computing environment.

The method obtains, at S10, the magnetic resonance image (MRI) and the body surface electrical potential measurement associated with the MRI. By "obtaining", it is meant any type of data acquisition that allows the retrieval or obtention of the MRI by the method.

Obtaining S10 the MRI and the body surface electrical potential measurement associated with the MRI may for example comprise downloading/retrieving (e.g. through a network) said MRI and said associated measurement from a (e.g. distant) memory or server or any non-volatile storage where these data have been stored further to a magnetic resonance imaging process that yielded the MRI and the associated measurement. The magnetic resonance imaging process may consist in any magnetic resonance imaging process also comprising, simultaneously to the imaging process, performing the body surface electrical potential measurement, for example with two or more surface electrodes.

Alternatively, the method may comprise performing this magnetic resonance imaging process, in which case the obtaining step S10 may consist in performing this process. For example, the method may send instructions to another computer system for performing magnetic resonance imaging acquisition and obtaining the MRI and the associated measurement in real time.

The obtained measurement may thus stem from measurements performed by two or more surface electrodes (i.e. placed on the body during the MRI process), as previously said, whether the obtaining S10 comprises performing these measurements (during a magnetic resonance imaging process) or retrieving them. The two or more surface electrodes placed on the body may be placed in any manner, e.g., close to the portion of the tissue captured by the MRI, so as to allow the obtention of the measurements. This is all known per se in the field of medical imaging.

The obtained MRI thus stems from a magnetic resonance imaging process, as discussed above, whether the obtaining S10 comprises performing this process or retrieving the MRI and measurements stemming from the process. As known in the field of medical imaging, an MRI is an image that stems from a magnetic resonance imaging process and that provide a 2D or 3D representation of the inside of a body of a patient (e.g. human patient) with a relatively high contrast resolution. The MRI in the method is an MRI of the portion of the tissue, and thus provides a 2D or 3D representation of the inside of this portion. The portion of the tissue is a part of a tissue of the body, said part being imaged by the MRI. The MRI is thus a 3D representation of this tissue part, thereby, the MRI may comprise 3D volumes or one or more 2D layer/slices arranged in 3D space representing the tissue. The tissue portion is an inside/inner portion of the body, e.g. that has a relatively homogeneous morphological and/or physiological properties, e.g., tissue of a given body part such as a given organ or a body part comprising a given artery or vein portion. The tissue may be directly below the skin of the patient or located more deeply within the patient's body.

The body surface electrical potential measurement is any measure of on the surface of the body of the electrical potential yielded by the electro-magnetic field created by the MRI system in surface at the portion. The measurement may be a single measurement or a collection/distribution of electrical potential values over one or more positions of the portion of the surface of the body, e.g., measured on the skin of the body. The measurement is associated with the MRI. By associated it is meant that the two objects (the measurement and the MRI) are related to each other. In specific, the measurement is a measurement, at the surface of the body *(e.g.* at the portion or near the portion) of the electrical potential induced by the electro-magnetic field created by the MRI system that has acquired the obtained MRI. The measurement and the MRI may thus be obtained, at S10 from a same body and/or at the same time. The measurement is at the surface of the body, near the portion or at the portion, that is on any part of the body surface (e.g. body skin) suitable for measuring the electrical potential yielded by the electro-magnetic field created by the MRI system in surface at the portion. The measurement may for be a distribution of electrical potential values each at a location of the patient's skin above or near the portion.

The obtained body surface electrical potential measurement stems from measurements performed by two or more surface electrodes placed on the body. For example, the measurements may be obtained at the same time while acquiring an MRI of the body. Indeed, when undergoing MRI acquisition, one may be equipped with two or more surface electrodes that are used for aligning the imaging process with other variables such as cardiac rhythm. The method may then acquire the measurements that stem from the magnetic fields applied during the MRI acquisition. Thereby, the method may obtain the measurements in a non-invasive manner, which improves material comfort to the body.

The method estimates, at S20, the distribution of conductivity on the portion of tissue based on the body surface electrical potential measurement and on the MRI. The conductivity is the electrical conductivity, but may simply be referred to as "the conductivity" in the disclosure. The distribution of conductivity on the portion of tissue may be a collection of one or more conductivity values at one or more space locations that are spatially distributed within the portion of the tissue, i.e. at its boundary and/or internal tissue, that is, the inner structure of the tissue comprised within its physiological and/or morphological borders. The estimation is based on the body surface electrical potential measurement and on the MRI, *i.e.* the method uses the MRI and the body surface electrical potential measurement to estimate the distribution of conductivity, for example by performing a suitable numerical method. The distribution of conductivity values represents how the tissue portion conducts electricity when subject to the electro-magnetic field created by the MRI system, which provides biological data about the inner structure of the tissue portion (i.e. how the conductivity is distributed on the tissue portion captures its inner biological and/or morphological structure). As known from the field of electromagnetism, the distribution of conductivity is indeed related to the body electrical potential that stems from the electro-magnetic field created by the MRI system, e.g., as described by Maxwell's equations. It is to be noted that the method has as input not the electrical potential *per se,* but only a body surface measurement thereof *(i.e.* electrical potential within the tissue portion is not known). However, the method takes into account/takes as input, concomitantly, the information provided by the MRI image (that is, morphological data representative of the portion of the tissue) and this surface electrical potential measurements to estimate the distribution of the conductivity in the portion of the tissue, that is the distribution of the conductivity both at the surface of the portion and inside the portion. That is, the method estimates the conductivity of the portion of tissue both at its surface and at its inner structure. The method may further obtain, e.g. concomitantly to the conductivity distribution, e.g. using the same numerical method, a distribution of the values of the electrical potential over the tissue portion, that is electrical potential values both inside the tissue portion and at its surface, the values at the surface matching, or tending to match, the measurement.

This results in that the method is able to obtain an accurate estimation of conductivity that respects the morphology of the tissue as well as the surface electrical potential measurement. Indeed, as the method takes into account the data representative of the portion of the tissue, the estimated distribution of conductivity respects the spatial distribution of the portion of tissue.

The mathematical framework of Maxwell's equations, illustrating the relationship between the body surface electrical potential measurements on the MRI and the distribution of conductivity is now explained.

Let $\Omega$ be a bounded domain in $R^3$ and $\partial\Omega$ be its boundary. The domain $\Omega$ is derived from the MRI. That is, a given element x belonging to the domain $\Omega$ (represented by 3D coordinates) is related to a location (e.g., 2D or 3D) of the MRI. In other words, the method may perform any kind of transformation that connects the MRI to the bounded domain. It is assumed hereinbelow that the conductor is nonmagnetic and isotropic. According to Faraday's law, a quasi-static current distribution is generated inside the conductor when applying low frequency time-varying magnetic fields. It is also assumed that these time-varying magnetic fields are sinusoidal of frequency $\omega$ (rad/s) and satisfying Maxwell's equations:

$$\nabla \times \mathbf{E} = -j\omega \mathbf{B} \qquad (1)$$

$$\nabla \times \mathbf{B} = \mu_0(\sigma + j\omega\epsilon)\mathbf{E} \qquad (2)$$

$$\nabla \cdot \mathbf{E} = \frac{\rho}{\epsilon} \qquad (3)$$

$$\nabla \cdot \mathbf{B} = 0, \qquad (4)$$

where $\sigma$ (S/m) is the electrical conductivity, $\mu_0$ is the vacuum permeability (H/m), $\varepsilon$ is the permittivity (F/m), $\rho$ is the charge density ($C/m^3$), E and B are respectively the electric field intensity and magnetic flux density. The continuity equation reads:

$$\nabla \cdot \mathbf{J} = -j\omega\rho \qquad (5)$$

where J is related to E by:

$$\mathbf{J} = (\sigma + j\omega\epsilon)\mathbf{E}. \qquad (6)$$

Equation (4) states that E is purely solenoidal. From the Helmholtz decomposition, one can introduce a magnetic vector potential A that satisfies:

$$\nabla \times \mathbf{A} = \mathbf{B}. \qquad (7)$$

The electrical potential $\phi$ (which is a scalar potential) can be introduced by inserting the magnetic flux expression of equation (7) into equation (1):

$$\mathbf{E} = -j\omega\mathbf{A} - \nabla\phi. \qquad (8)$$

Since there is no charge density in the object, then $\rho = 0$, thus leading to the continuity equation:

$$\nabla \cdot ((\sigma + j\omega\epsilon)\mathbf{E}) = 0. \qquad (9)$$

Substituting equation (8) into (9) yields:

$$\nabla \cdot ((\sigma + j\omega\epsilon)\nabla\phi) = -j\omega\nabla \cdot ((\sigma + j\omega\epsilon)\mathbf{A}). \qquad (10)$$

Equation (10) links the conductivity $\sigma$ and the potential $\phi$.

The estimation of the distribution of conductivity, at S20, may comprise obtaining a solution of an inverse problem over the portion of tissue. In other words, the method retrieves the distribution of conductivity given the body surface electric potential measurements and the MRI as the solution of an inverse problem. The solution of the inverse problem consists of a distribution of one or more conductivity values satisfying a system of equations. That is, the method may determine the conductivity values that correspond to *(i.e.* as captured by the system of equations) the body surface electrical potential measurement on the portion of the tissue represented by the MRI. The system of equations is derived from Maxwell's equations associated with the body surface electrical potential measurement. By "derived" it is meant that the system of equations may be an alternative or approximate form of Maxwell's equations that relate the conductivity induced by the electro-magnetic field created by the MRI and the electrical potential induced by the electro-magnetic field. In other words, the solution of the system of equations is substantially an approximation and/or a modified formulation (e.g., up-to an uncertainty when the system of equations is of an approximate form, or completely equivalent when the system of equations is of an alternative form) to the solution of Maxwell's equation (which describe the physical laws of propagation of electrical and magnetic fields) where the electrical potential matched the body surface electrical potential measurement at the domain's boundary (i.e. the body surface). The restriction up-to which the system of equations is derived may comprise physical principles, for example, constraining the solution of Maxwell's equations to the case where displacement currents are negligible, or constraining the solution to the case that no electrical current can flow from the boundary of the tissue into non-tissue portions (e.g., air).

The derivation (i.e. obtention, calculation) of the system of equations from Maxwell's equations is now explained. This derivation comprises the derivation of equations (1) to (10) explained previously, and further computation steps which are now discussed.

Since it is considered the application of low frequency magnetic fields, and further considering body tissue conductivity values ranging from $10^{-1}$ to 1 S/m, it follows that σ » $\omega\varepsilon$. Hence, the displacement currents are negligible and so equation (10) admits a simplification to the governing equation of the direct problem:

$$\nabla \cdot (\sigma \nabla \phi) = -j\omega \nabla \cdot (\sigma \mathbf{A})\,. \qquad (11)$$

Moreover, on the boundaries, no current can flow from the object into the air. In other words, J · n = 0, with n being the normal component of ∂Ω. This leads to the Neuman boundary condition (of the direct problem) of the form:

$$\frac{\partial \phi}{\partial n} = -j\omega \mathbf{A} \cdot \mathbf{n}\,. \qquad (12)$$

In practice, equations (11) and (12) need to be simultaneously solved along with their coupled equation for the magnetic potential:

$$\nabla^2 \mathbf{A_s} = -\mu_0 (\sigma + j\omega\epsilon)(\nabla\phi + j\omega(\mathbf{A_p} + \mathbf{A_s}))\,. \qquad (13)$$

The equations may be further simplified as follows. Under the assumption that, for a volume object of size L, the condition $\omega\mu_0\sigma \ll \dfrac{1}{\mathrm{L}^2}$ holds, the magnetic vector potential A can be approximated by the primary potential vector $A_P$.

One $A_P$ is calculated, the direct problem can be simplified and restricted to a system of two equations involving only electric potentials of the form:

$$\nabla \cdot (\sigma \nabla \phi) = -j\omega \nabla \cdot (\sigma \mathbf{A_p}) \qquad (14)$$

$$\frac{\partial \phi}{\partial n} = -j\omega \mathbf{A_p} \cdot \mathbf{n}\,. \qquad (15)$$

Equations (14)-(15) form the system of equations derived from Maxwell's equations associated with the body surface electrical potential measurement. The equations (14)-(15) state the direct problem, whenever the values of σ and $\phi$ are known. The inverse problem consists on finding (or estimating) values of σ and $\phi$ such that equations (14)-(15) hold.

Using this system improves the accuracy of the estimation. Indeed, the method thereby obtains the estimated the distribution of conductivity from the system of equations describing the physical laws governing the physical phenomenon linking the propagation of the electrical and magnetic fields with body surface electric potential measurements. This ensures that the estimated distribution is accurate enough to represent the distribution on the surface of the portion of tissue and in its interior/inner structure. Indeed, the system of equations ensures that the estimated distribution of conductivity is representative of the physical flow of electrical current on the portion of the tissue, e.g., is a flow that respects the boundaries and internal structure of the tissue, without artifacts such as currents going to non-tissue portions such as air. Furthermore, using the system of equations allows the method to use any suitable numerical method for solving said inverse problem associated with the system, for example a finite element method.

Obtaining the solution of the inverse problem comprises learning a neural network based on the obtained MRI and the obtained body surface electrical potential measurement, and using the learnt neural network to obtain the solution of the inverse problem.

The neural network is a computer-implemented/computerized data structure that is a set of layers of neurons defined by two features: topology and parameters (also known as weights). The topology may be a system of connected computing units. The weights may be numerical coefficients which are assigned to each connection. Each computing unit may output a weighted combination of a corresponding input numerical data and sends this result to neighboring computing units according to the connections. The neural network may comprise initial units which are fed by input data (that is, the obtained MRI and the obtained body surface electrical potential measurement) and terminal units which yields the output result (that is, the distribution of connectivity). The topology of the network, in combination with the weights assigned to each connection, may establishes the flow of data from the initial units all the way down to the terminal units. Initially, the learning may provide no importance to the weights' values. For instance, the weights may be randomly preset.. The learning of the neural network may comprise adjusting at least part of the weight's values, and thus adjust its importance.

The neural network is configured for estimating the distribution of conductivity on the portion of tissue as an approximated solution of the system of equations. That is, learning may adjust at least part of the weight's values so that the output of the neural network approximates the solution of the system of equations. In other words the neural network is trained so that the system of equations is satisfied by the estimated distribution of conductivity on the portion of tissue. Thus, the neural network is trained so that its output respects the physical laws (set by the system of equations) which describe the physical phenomenon linking the propagation of the electrical and magnetic fields on the portion of the tissue with body surface electric potential measurements.

Using the learnt neural network to obtain the solution of the inverse problem comprises feeding the obtained MRI and the obtained body surface electrical potential measurement as input to the learnt neural network, which then outputs the estimated distribution of conductivity. As the neural network is configured for estimating the distribution of conductivity on the portion of tissue as an approximated solution of the system of equations, the method obtains an approximated solution of the inverse problem.

The method thus leverages from the use of neural networks for obtaining an accurate estimation of the distribution of conductivity. Indeed, as the neural network is configured for estimating the distribution of conductivity on the portion of the tissue, its training/learning integrates the morphology of the portion of the tissue in a more efficient manner. Indeed, this is all thanks to the fact that the training adjusts the weights so as to as to approximate the system of equations, which respect the physical link between the propagation of the electrical and magnetic fields on the portion of the tissue with body surface electric potential measurements. The situation is also improved compared to existing methods based on solving finite element problems. Indeed, on one hand, finite element methods suffer from scalability problems which render the solution of an inverse problem intractable as soon as the resolution of the mesh of the corresponding finite element model grows. On the other hand, finite element methods also suffer from high memory requirements when increasing the resolution of the discretization performed by finite element methods. This is particularly disadvantageous whenever there is the need to increase detail of the estimation.. In contrast, the method is independent of the resolution of the mesh, and thus does not suffer from said drawbacks.

The learning comprises minimizing a loss. The loss comprises a term that depends increasingly on residual functions corresponding to the system of equations. By "depends increasingly", it is meant that the value (e.g., real value) of the term increases as the value of the residual function increases. The residual functions quantify an extent to which the system of equations is satisfied by an estimated distribution of conductivity on the portion of tissue. Hence, by minimizing the loss, the method penalizes a non-agreement of the estimated distribution of conductivity on the portion of tissue with respect to the system of equations. Thereby, as the term depends increasingly on the residual functions, the learning adjusts the weights so that value of the term decreases so as to increase the extent to which the system of equations is satisfied by an estimated distribution of conductivity on the portion of tissue.

This results in that the method provides an accurate estimation of the distribution of conductivity, without needing to solve explicitly the system of equations, which may be intractable due to the fact that the equations are derived from Maxwell's equations, and without the need to solve a finite element problem. This improves greatly the computational efficiency: the main computational task is performed during the training (in an off-line stage). The training consists in solving the inverse problem and (e.g. in an on-line stage, that is, in a medical application) reconstructing the distribution of conductivity (which may be later provided as an image). The speed of the training, that is, the time needed for the convergence of the minimization of the loss, is of only some minutes, which is acceptable and reasonable for medical applications. Notably, the speed of the learning is faster than FEM methods, which have significant computational overhead due to the dependence on the mesh resolution.

The neural network may be further configured for estimating an electrical potential of the tissue. That is, the neural network is trained to estimate the electrical potential on the whole tissue portion, i.e. both inside the portion and at its surface. Since, the surface electrical potential is at least partially known, this means that the neural network finds a distribution of the electrical potential values that complete and matches the known surface electrical potential. The system of equations link the conductivity of the tissue and the surface electrical potential. As the method estimates the conductivity, the method may as well obtain an estimation of the potential as part of the solution of the inverse problem. This may be seen as a sort of interpolation of the body surface electric potential measurements. To do so, the loss further comprises in this case a term that penalizes non-respect of the body surface electrical potential measurement by an estimated electrical potential of the tissue. This term evaluates an extent to which the electrical potential values as estimated by the neural network matches the measurement at the body surface.

Thus, in conjunction with the other terms in the loss the method is able to estimate the distribution of conductivity and the electrical potential over the body portion, that is inside the portion and at a surface thereof, in a manner that makes the estimation consistent with the surface measurement provided at S10. This further improves reliability and accuracy of the estimation.

The loss may further comprise a term that penalizes a non-respect of prior tissue segmentation data by a gradient of the estimated distribution of conductivity. The prior tissue segmentation data is prior information regarding the inner structure of the body, and that is indicative of a segmentation of the portion of the tissue with respect to the rest of the body. The prior tissue segmentation may be or stem for a segmentation of the IRM. The prior tissue segmentation data may for example be a space or surface (e.g. partial) segmentation of the MRI in segments each representing a tissue sub portion of the tissue portion. Such a segmentation may be obtained (e.g. at an initial stage of the method or after performing the MRI imaging process) by any suitable known segmentation method. In the learning, the method penalizes a non-respect of this data by a gradient of the estimated distribution of conductivity. The term may for example depend increasingly on the conductivity gradient (i.e. the gradient of the estimated conductivity) on portions of the tissue segmentation where the underlying segmentation function has a zero gradient, that is on portions corresponding to a same biological and/or morphological part where the conductivity is expected (i.e. supposed) to be constant or at least not to present significant variations. Thus, the term enforces that the conductivity should be constant on portions where the segmentation gradient is zero (because the prior segmentation captures that this ought to be the case in reality) and allows the conductivity to present variations (e.g. smooth variations)

This improves the accuracy of the method. Indeed, the learning also regularizes the approximation of the solution of the system of equations by keeping the gradient of the estimated distribution of conductivity as low as possible in the regions where the conductivity is supposed to be homogeneous (i.e., regular), while allowing jumps along discontinuities.

An example of the loss is now discussed.

In the following, let $\Omega$ denote a bounded domain and derived from the MRI. In other words, the domain may be a subset of values identified by 3D coordinates, e.g., by x-y-z coordinates. The boundary of the domain $\Omega$ may be denoted as $\partial\Omega$. Also, x may denote a real-valued vector belonging to a domain $\Omega$. As discussed previously, the domain $\Omega$ is derived from the MRI, and thus x is related to a location (e.g., 2D or 3D) of the MRI.

Let $\sigma(x)$ be a function that represents the distribution of conductivity on the portion of the tissue. That is, $\sigma(x)$ may be a real-valued function taking values x on the domain $\Omega$ and outputting a respective conductivity value at position x. As x is related to a location of the MRI, the output of $\sigma(x)$, corresponds to a value of the conductivity on said location of the MRI. Let $\phi(x)$ be a function that represents the electrical potential. That is, $\phi(x)$ may be a real-valued function taking values on the domain $\Omega$ and outputting the electrical potential at position x. As x is related to a location of the MRI, the output of $\sigma(x)$, corresponds to a value of the conductivity on said location of the MRI.

The loss is of the type:

$$\mathcal{L}\left(\boldsymbol{\theta}_{\sigma}, \boldsymbol{\theta}_{\phi}\right) = \mathcal{L}_1(X_1) + \lambda_2 \mathcal{L}_2(X_2) + \lambda_M \mathcal{L}_M(X_M) + \lambda_R \mathcal{L}_R(\boldsymbol{\theta}_{\sigma}) + \lambda_p \mathcal{L}_P(X_P)$$

By "of the type", it is meant that the loss $\mathcal{L}$ respects the above formula, e.g. up to a certain scaling and/or up to the presence of regularization terms.

The variables $\theta_{\sigma}$ and $\theta_{\phi}$ represent parameters of the neural network. In other words, the variables $\theta_{\sigma}$ and $\theta_{\phi}$ comprise weights of the neural network which are adjusted during the training so as to minimize the loss.

The terms $\mathcal{L}_1(X_1)$ and $\lambda_2 \mathcal{L}_2(X_2)$ in the loss are terms that depend increasingly on residual functions corresponding to the system of equations.

The system of equations may be denoted as:

$$\nabla \cdot (\sigma \nabla \phi) = -j\omega \nabla \cdot (\sigma \boldsymbol{A}),$$

$$\frac{\partial \phi}{\partial n} = -j\omega \boldsymbol{A}.$$

The terms $j$, $\omega$, $n$ and $A$ are respectively the current density, angular frequency, the normal vector and the magnetic vector. The term $\boldsymbol{A}$ may also be approximated by the term $\boldsymbol{A_P}$, which denotes the primary potential vector, and thus may be used interchangeably in the definitions below. The solution of the system of equations satisfies approximately Maxwell's equations, yet it only involves electric potentials. Details on its derivation are provided later.

The term $\mathcal{L}_1(X_1)$ is of the form (e.g. may be exactly) $\mathcal{L}_1(X_1) = \frac{1}{N_1} \sum_{x_i \in X_1} R_1(\boldsymbol{x_i})^2$. $N_1$ is a natural number

and $X_1$ is a set of $N_1$ points of the domain $\Omega$. The set of $N_1$ points may be sampled (obtained) from the domain, e.g., as a subset of $\Omega$. The set may be sampled in any manner, e.g., randomly. $R_1(x)$ is a residual function of the type $R_1(x) := \nabla \cdot (\sigma(x) \nabla \phi(x)) + j\omega \nabla \cdot (\sigma(x) A_P(x))$. The residual function $R_1(x)$ quantifies the extent to which the equation $\nabla \cdot (\sigma \nabla \phi) = -j\omega \nabla \cdot (\sigma A)$ is satisfied by the estimated distribution of conductivity $\sigma(x)$ and the estimated potential $\phi(x)$.

The term $\lambda_2 \mathcal{L}_2(X_2)$ is of the form (e.g. may be exactly) $\lambda_{22}(X_2) = \dfrac{\lambda_2}{N_2} \sum_{x_i \in X_2} R_2(x_i)^2$ , $N_2$ may be a natural number and $X_2$ is a set of $N_2$ points on a boundary of the domain $\Omega$ (e.g., sampled from the boundary), $\lambda_2$ is an adjustable non-negative and non-zero coefficient, and $R_2(x)$ being another residual function of the type

$$R_2(x) := \frac{\partial \phi}{\partial n}(x) + j\omega A_P(x) \cdot n$$

. n. The residual function $R_2(x)$ quantifies the extent to which the equation

$$\frac{\partial \phi}{\partial n} = -j\omega A$$

(also called Neumann boundary condition) is satisfied by the estimated distribution of conductivity $\sigma(x)$ and the estimated potential $\phi(x)$.

The term $\lambda_M \mathcal{L}_M(X_M)$ penalizes non-respect of the body surface electrical potential measurement by the estimated electrical potential $\phi(x)$ of the tissue. The term $\lambda_M \mathcal{L}_M(X_M)$ is of the form (e.g. may be exactly) $\lambda_M \mathcal{L}_M(X_M) =$

$$\frac{\lambda_M}{N_M} \sum_{x_i \in X_M} (\phi(x_i) - \Phi_i)^2$$

, $N_M$ is a natural number, $X_M$ is a set of $N_M$ points of the boundary of the domain $\Omega$ (e.g., sampled from the boundary), and $\lambda_M$ is an adjustable non-negative (e.g. non-zero, or, alternatively, possibly taking the value zero) coefficient. The values $\Phi_i$ of $\phi(x_i)$ $x_i \in X_M$ form the obtained body surface electrical potential measurement, i.e. the obtained measurements consists in the distribution of the values $\Phi_i$.

The term $\lambda_R \mathcal{L}_R(\theta_\sigma)$ is an L2-regularization term of the body surface electrical potential measurement. The term $\lambda_R \mathcal{L}_R(\theta_\sigma)$ is of the type $\lambda_R \mathcal{L}_R(\theta_\sigma) = \lambda_R \sum_{\Theta_i \in \Theta_\phi} \Theta_i^2$. $\lambda_R$ is an adjustable non-negative (possibly zero) coefficient.

$\mathcal{L}_P(X_P)$ is a term that penalizes a non-respect of prior tissue segmentation data (denoted as S) by the gradient of the estimated distribution of conductivity $\nabla\sigma(x)$. The term $\mathcal{L}_P(X_P)$ is of the type

$$\lambda_p \mathcal{L}_P(X_P) = \frac{\lambda_p}{N_P} \sum_{x_i \in X_P} \|\nabla\sigma(x)\| \cdot P(x_i)$$

.

$P(x_i)$ is a function of the type $P(x_i) = \begin{cases} 1 & \text{if } \|\nabla S(x_i)\| = 0 \\ 0 & \text{otherwise} \end{cases}$ . The term $S(x_i)$ denotes an element of prior tissue segmentation data S. $N_P$ is a natural number, $X_P$ is a set of $N_P$ points, and $\lambda_p$ is an adjustable non-negative coefficient. In implementations, $N_P$ may be chosen as being equal to $N_1$, respectively $X_P$ may be chosen as being equal to $X_1$.

The terms $\lambda_2$, $\lambda_M$, $\lambda_R$ and $\lambda_p$ may be adjustable non-negative coefficients. In other words, the terms are positive coefficients, and some of the coefficients may be zero. For example, at least one term may be strictly positive, e.g., the coefficient $\lambda_2$ may be strictly positive, while each other term may be either strictly positive or zero. Thereby, the loss may focus on individual aspects of the learning. Indeed, the method may order the terms $\lambda_2$, $\lambda_M$, $\lambda_R$ and $\lambda_p$ in any manner so as to provide more importance to the learning of a corresponding term. Alternatively, the method may give to all the terms the same importance. In examples, all the terms are strictly positive.

The neural network may comprise a first sub-network and a second sub-network. The first sub-network may be a sub-system of connected computing units of the neural network, with its corresponding parameters. The second sub-network may be another sub-system of connected computing units of the neural network, with its corresponding parameters. The first sub-network and the second sub-network may be interconnected according to a predetermined connection topology. The first sub-network comprises first parameters to be set during the learning. That is, initially (i.e. before the learning begins), the first parameters may be preset in any manner, e.g., randomly and the learning of the neural network then adjusts their values. The second sub-network may comprise second parameters to remain fixed during the learning. The second parameters may be obtained in any manner, e.g., being obtained randomly or retrieved by any means.

The method thus allows to test several initial configurations, so that the method provides information on how changing in initial configuration influences the result. Indeed, the first parameters are preset in any manner initially and adjusted during the training, while the second parameters are fixed during the learning, however possibly randomly sampled. Hence, the variability of the second parameters on each training provides a quantification of uncertainty on the estimated distribution of conductivity.

The learning of the neural network may be iterated. The method may perform, e.g., a predetermined number of iterations (e.g., one or more) and performing the learning again for each iteration. Each iteration comprises modifying the second parameters of the second sub-network. That is, each iteration may fix independently the second parameters prior to the learning. Hence, on a given iteration, the learning adjusts the first parameters while the newly set second parameters remain fixed. The learning may compute an estimation as a set of predictions. Thus, the method is able to generate rich and varied data that provides more information than a single estimation. The set of predictions is suitable for providing the prediction confidence or uncertainty of the estimation.

The method may further comprise computing a mean of the estimated distribution of conductivity obtained from each iteration of the learning. Hence, the method provides a prediction confidence, by providing the mean of the estimated distribution of conductivity. The method may also comprise computing a variance of the distributions. The variance corresponds to the uncertainty of the distributions. Thus, the method also provides information that quantifies uncertain estimations, which affect the confidence of the estimation.

The method may further comprise performing a modified body surface electrical potential measurement based in the uncertainty. The method may, for example, modify the parameters of the neural network such as modifying the location of the potential measurement to be estimated. The method may also comprise re-learning the neural network based on the modified measurement. The method may, e.g., modify the location of the potential measurement on locations of the MRI where the estimated distribution of conductivity has an uncertainty with value larger than a predetermined threshold. The method may then re-learn the neural network. As the learning minimizes the loss, the method reduces the uncertainty of the region where the modified measurement is located. This improves the accuracy of the estimation, as the learning takes into account regions of uncertainty to improve the confidence of the estimation where the uncertainty is the highest, e.g., above a predetermined threshold.

A particularly efficient implementation of the loss is now discussed.

Let x $\in \Omega$. The aim of the inverse problem is to retrieve the conductivity values $\sigma(\boldsymbol{x})$ given body surface potential measurements $\phi(x)$ on a restricted set of points on $\partial\Omega$.

To solve the inverse problem, the loss minimizes two residual functions, which are defined from equations (14) and (15). The residual functions write:

$$R_1(\mathbf{x}) := \nabla \cdot (\sigma(\mathbf{x})\nabla\phi(\mathbf{x})) + j\omega\nabla \cdot (\sigma(\mathbf{x})\mathbf{A_p}(\mathbf{x})) \qquad (16)$$

$$R_2(\mathbf{x}) := \frac{\partial\phi}{\partial n}(\mathbf{x}) + j\omega\mathbf{A_p}(\mathbf{x}) \cdot \mathbf{n}. \qquad (17)$$

The conductivity values $\sigma(\boldsymbol{x})$ and the body surface potential measurements $\phi(x)$ may be represented in the neural network with two different sub-networks $f_\sigma$ and $f_\phi$. The variables write:

$$\sigma(\mathbf{x}) \approx f_\sigma(\mathbf{x}, \Theta_\sigma) \qquad (18)$$

$$\phi(\mathbf{x}) \approx f_\phi(\mathbf{x}, \Theta_\phi). \qquad (19)$$

A set $X_1$ of $N_1$ points is randomly drawn from the domain $\Omega$. Those points are the collocation points where the residual $R_1(\boldsymbol{x})$ is minimized. In other words, the aim of the loss is to enforce equation (14) on that set. Another set $X_2$ of $N_2$ points is randomly drawn from the domain $\partial\Omega$. The set $X_2$ is used to minimize the residual $R_2(x)$. In other words, the loss enforces the Neumann boundary condition (15) on $\partial\Omega$.

Additionally, $N_M$ measurement points are drawn from $\partial\Omega$. to form the set $X_M$ where the values $\Phi_i$ of $\phi(x_i)$, $x_i \in X_M$ are known. Data consistency will assure the solution is in line with the measurements performed at these points.

Finally, the sub-networks $f_\sigma$ and $f_\phi$ are jointly trained by minimizing the following loss function:

$$\mathcal{L}(\boldsymbol{\theta}_{\sigma}, \boldsymbol{\theta}_{\phi}) = \mathcal{L}_1(X_1) + \lambda_2 \mathcal{L}_2(X_2) + \lambda_M \mathcal{L}_M(X_M) + \lambda_R \mathcal{L}_R(\boldsymbol{\theta}_{\sigma}) + \lambda_p \mathcal{L}_P(X_P)$$

$$= \frac{1}{N_1} \sum_{x_i \in X_1} R_1(\boldsymbol{x_i})^2 + \frac{\lambda_2}{N_2} \sum_{x_i \in X_2} R_2(\boldsymbol{x_i})^2 + \frac{\lambda_M}{N_M} \sum_{x_i \in X_M} (\phi(\boldsymbol{x_i}) - \Phi_i)^2$$

$$+ \lambda_R \sum_{\theta_i \in \boldsymbol{\theta}_{\phi}} \theta_i{}^2 + \frac{\lambda_p}{N_P} \sum_{x_i \in X_P} \|\nabla\sigma(\boldsymbol{x})\| \cdot P(\boldsymbol{x_i}). \qquad (20)$$

$\mathcal{L}_1(X_1)$ and $\mathcal{L}_2(X_2)$ and $\mathcal{L}_M(X_M)$ are minimum square error (MSE) Losses aiming at minimizing $R_1(x)$ at $X_1$, $R_2(\boldsymbol{x})$ at $X_2$, and minimizing the difference between estimated potentials and real (i.e., obtained) body surface potential measurements on $X_M$. $\mathcal{L}_R$ is an L2 regularization term of the parameters of the sub-network $f_\phi$. $\mathcal{L}_P(X_P)$ is the term that penalizes a non-respect of prior tissue segmentation data by the gradient of the estimated distribution of conductivity. The different coefficients $\lambda_2$, $\lambda_M$, $\lambda_R$ and $\lambda_P$ are adjusted so as to give more or less importance to a particular term. The terms may be set empirically.

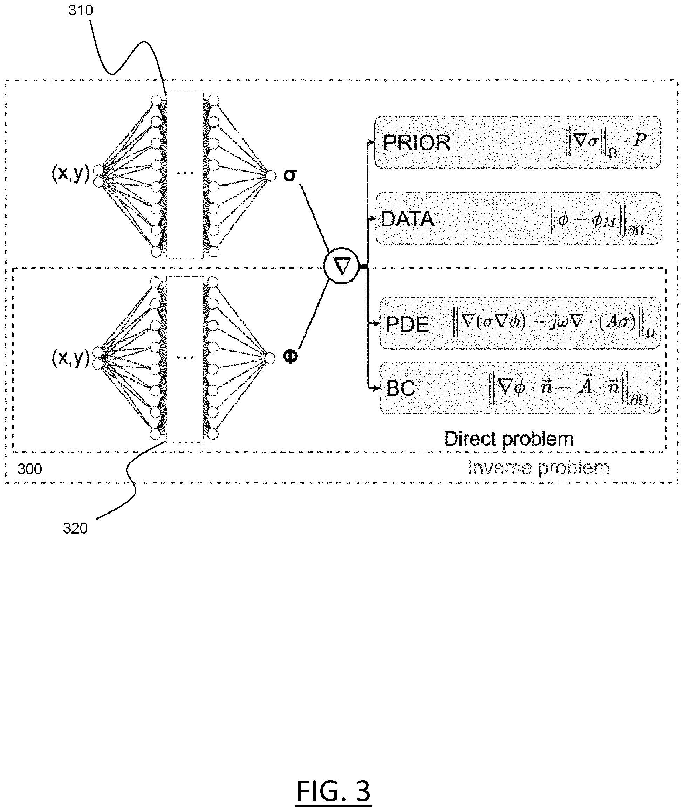

Reference is made to FIG. 3, illustrating the neural network. The neural network 300, used for solving the inverse problem and thereby estimating the distribution of conductivity, is divided into a first sub-network 310 and a second sub-network 320. The computing units of the first sub-network 310 activation functions Relu. The computing units of the second sub-network 320 comprise activation functions tanh. Both of the sub-networks 310 and 320 are fully connected neural networks. The first sub-network 310 takes as input (x,y)-coordinates of pixels and outputs the conductivity values $\sigma(\boldsymbol{x})$. The second sub-network 320 also takes as input (x,y)-coordinates of pixels and outputs body surface potential measurements $\phi(\boldsymbol{x})$.

Aspects of the learning of the neural network are now discussed.

The neural network may provide prediction confidence and uncertainty maps (for image segmentation or reconstruction) for the estimation of conductivity, so that the end-user (clinicians) can focus on critical or uncertain cases. This improves the confidence of clinicians on the estimation, in contrast with only providing a single estimation.

The neural network may incorporate a technique to estimate uncertainty of the estimations by the neural network predictions. The technique is based on randomized prior functions. Each of the first sub-network and the second sub-network is divided into a sum of two networks (with the same architecture and designed), such that:

$$\sigma(\mathrm{x}) \approx f_\sigma(\mathrm{x}, \Theta_\sigma) + f_\sigma(\mathrm{x}, \tilde{\Theta}_\sigma) \qquad (21)$$

$$\phi(\mathrm{x}) \approx f_\phi(\mathrm{x}, \Theta_\phi) + f_\phi(\mathrm{x}, \tilde{\Theta}_\phi). \qquad (22)$$

The parameters $\tilde{\theta}_\sigma$ and $\tilde{\theta}_\phi$ are randomly drawn from a Gaussian prior distribution, and remain fixed during the training. Each individual estimation (as a result of each training) may be seen as drawing a sample from a posterior distribution $p(\theta|D)$. Solving the problem then consists in training the neural networks (with multiple drawn samples) on the same input data. The final prediction may be computed from the mean of the set of predictions, while the uncertainty is computed from the variance of the set of predictions.

Further aspects of the learning are now discussed.

The method may incorporate an active learning technique. Given initial sets of measurements and measurement locations, active learning consists in performing an iterative process, in which the learning determines, for each iteration, an optimal location (on the MRI) for the next set of measurements. This iterative process leads to a more robust solution, i.e. by decreasing uncertainty. To achieve this, the method may incorporate computing the entropy of the solution on each iteration. The method may determine the location of a new measurement (projected onto the boundary of the domain $\Omega$) at the location where the variance (as computed by the entropy) is the highest.

This aspect of the learning is critical in biomedical applications. Indeed, the optimal placement of measurement points and sensors is a recurring problem in biomedical applications due to the huge variability of the body's morphotype. As the active learning automatically determines the optimal sensor placement for each patient, this improves the accuracy of the solution of the inverse problem as it is adapted to the body's morphotype of the subject.

Experiments and results of the experiments are now discussed.

**Experiments**

It is now described the experiments used for analyzing the effectiveness of the method. Each experiment description will be accompanied by how the hyperparameters of the PINN are set.

The experimental testbeds described below include: A) solving the forward problem with an analytical solution, B) performing a simulation of a more realistic forward problem, C) solving the inverse problem with the proposed method, D) performing a numerical quantification of the uncertainty, and E) applying the active learning scheme, for the automated placement of the electrodes providing most information.

**A. Forward problem, with analytical solution**

The first experiment consists of a simplistic experiment, by computing the analytical (exact) solution. This experiment allows to assess the proposed solution ability to accurately solve this electromagnetic problem, while compared its performance with the state-of-the-art FEM method.

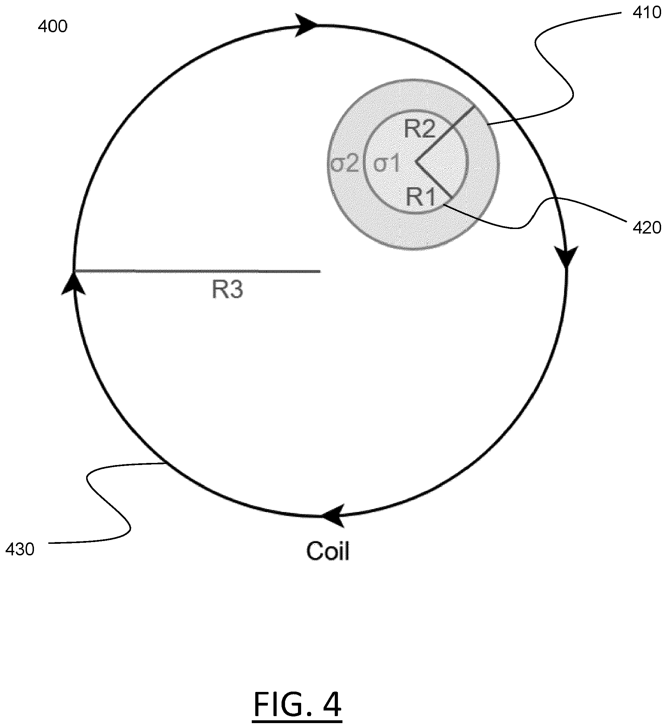

Reference is made to FIG. 4, depicting the configuration of the simulated environment. The configuration consists of two concentric circles and with different conductivity values. This configuration (also called phantom) is subject to a magnetic field from a simple circular coil 430. A circular discontinuity 420 of conductivity $\sigma_1$ of radius $R_1$ was concentrically introduced into a disc 410 of conductivity $\sigma_2$ and of radius $R_2$. The circular coil 430 of radius $R_3$ driving a current or radial frequency $\omega$ was simulated and its theoretical magnetic potential field calculated numerically using Biot-Savart's law.

In the forward problem, conductivity values are known and the potentials on the surface of the biggest circle are to be estimated. The analytical solution of this problem is given by the equation:

$$0 < r < R_1 :$$

$$\phi_1(r, \theta) = \sum_{m=1}^{\infty} r^m (e_m cos(m\theta) + f_m sin(m\theta)) \quad (23)$$

$$R_1 < r < R_2 :$$

$$\phi_2(r, \theta) = \sum_{m=1}^{\infty} \left( (r^m a_m + r^{-m} b_m) cos(m\theta) \right.$$
$$\left. + (r^m c_m + r^{-m} d_m) sin(m\theta) \right) \quad (24)$$

The analytical solution is then compared to the estimation provided by the neural network and a state of the art FEM method.

For testing the method, the experiment randomly generates $N_1$ = 1000 collocation points and $N_2$ = 300, so that $X_1, X_2$ and $X_M$ were randomly drawn from a uniform distribution inside the domains of interest. The networks were trained over 40,000 epochs using the ADAM optimizer with a learning rate of 0.001. The tests adopts a full-batch optimization scheme.

The state of the art FEM method was run on a 125,000 nodes mesh using the Fenics library. Both the proposed network and the FEM estimations were compared against the analytical solution by using the mean absolute error (MAE). Ten different configurations were simulated where $\sigma_1$, $\sigma_2$ and $R_1$ took random values within realistic physiological ranges. Since the potentials are defined up to a constant, $\omega$ and the potential-maps minimum were fixed so that the analytical solution ranges between 0 and 1V.

**B. Realistic forward problem simulation**

A more realistic simulation of the forward problem has been generated to further test the proposed technique. Electrical currents were induced by simulated MRI gradient coils.

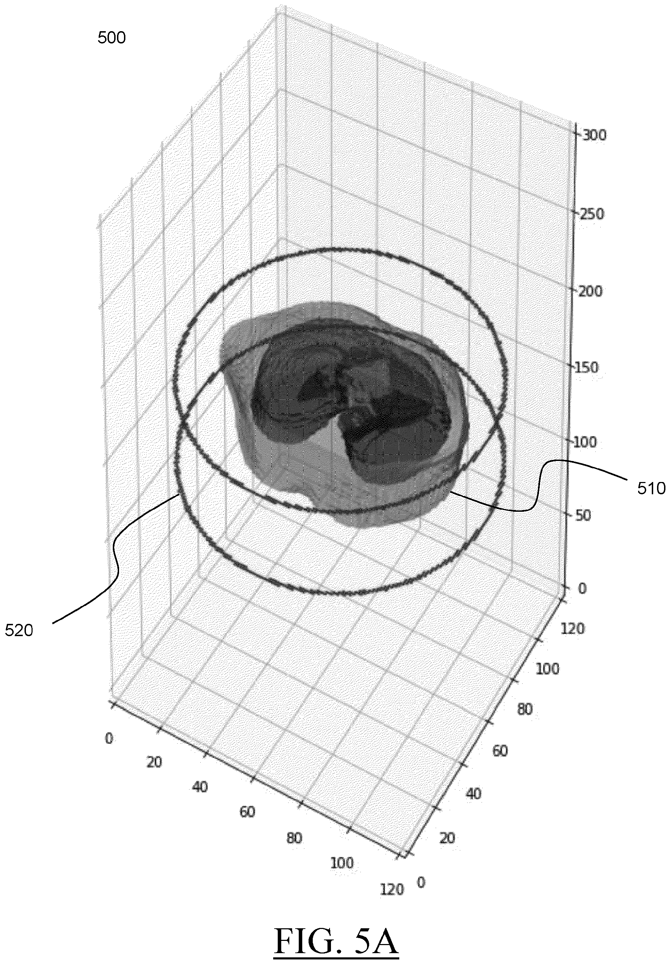

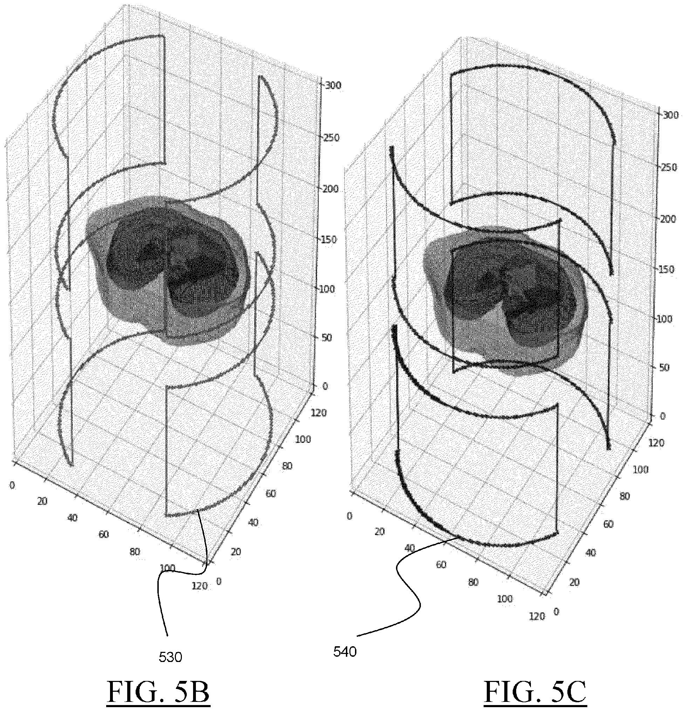

Reference is made to FIG.s 5A to 5C, illustrating simulated MRI gradient coils. FIG. 5A shows an MRI 500 with a portion of tissue 510. The experimental setup places, on the MRI 500, a simulated MRI gradient coil 520 around the portion of tissue 510. The experiment also places different MRI gradient coils to improve the variability of the induced electrical currents. FIG.s 5B and 5C show the same MRI, although with different simulated MRI gradient coils 530, and 540. The varying magnetic vector potential **A** is derived from MRI coils. Sinusoidal gradient pulses are played independently on the different coils.

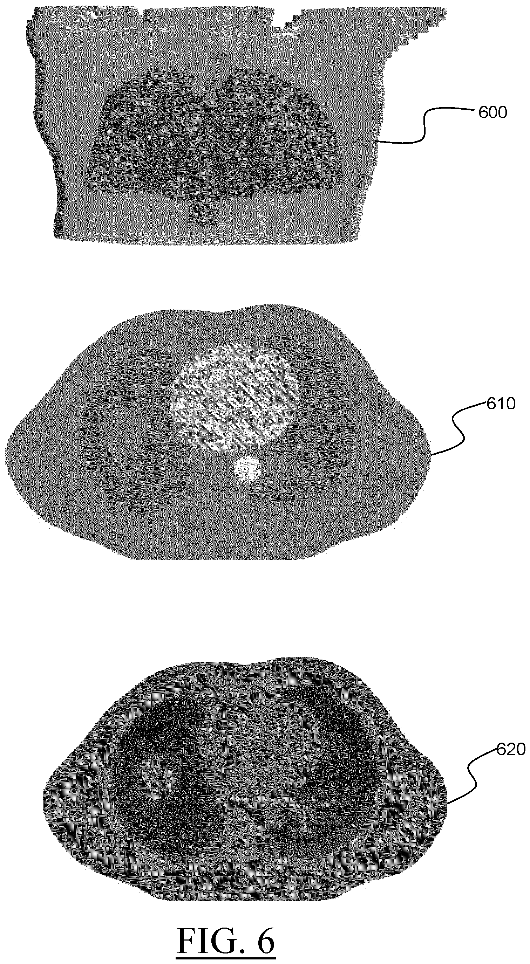

Reference is made to FIG. 6, illustrating a real torso shape 600 obtained from the SegThor database and corresponding segmentations 610 and 620. The segmentations 610 and 620 are used as a conductivity mask. The segmentation is performed via thresholding methods.

Conductivity values are assigned to the different segmented regions according with standard tissue conductivity values. The models are placed in the center of the simulated MR device and are discretized so that each pixel represents a cm2 area. A Gaussian filter is applied to smooth the conductivity images.

The resulting potential distributions are estimated after inducing currents with each of the three coils and compared to the FEM solutions. The analytical solution being unknown on such a configuration, the MAE between the FEM estimation and the PINN solution is used to verify that the MAE remained in the same range as the forward problem seen in the previous section. Thus the coils' amperage and pulses frequency were set so the FEM solution's range is 0-1 as before for a fair comparison.

## C. Inverse problem

Simulations of the inverse problem were run using the same configuration as described in the previous section. Referring back to FIG.s 5A to 5C, the currents running through the coils 520 to540 are set to mimic real imaging conditions. Electrodes were randomly positioned across the torso surface and the potentials were propagated and estimated using the solutions presented in the previous section. Those potentials are then used to train the inverse network following the loss of equation (20). Since there are three available coils, the inverse problem consists in finding the conductivity distribution that simultaneously solves the direct problem for the three different coils 520 to540.

In the scope of the method, it is assumed that in addition to the potential measurements, one has also access to MRI images and therefore a plausible conductivity segmentation. Prior information regarding the inner structure of the body to image can be added to the inverse problem optimization scheme in the form of the additional loss term $\mathcal{L}_P(X_P)$ found in Eq. 20 (with $\lambda_p$ set to 1):

$$\mathcal{L}_P = \frac{1}{N_P} \sum_{\mathbf{x}_i \in X_P} \|\nabla \sigma(x_i)\|.P(x_i) \qquad (25)$$

Where $P(x_i)$ is a prior segmentation information. Given a segmentation map of an MRI $S$, $P(x_i)$ is defined as follows:

$$P(x_i) = \begin{cases} 1 & \text{if } \|\nabla S(x_i)\| = 0 \\ 0 & \text{otherwise} \end{cases} . \qquad (26)$$

The goal of this additional loss term is to regularize the solution by keeping the conductivity gradient as low as possible in the regions where the conductivity is supposed to be homogeneous, while allowing jumps along the discontinuities. This can be seen as a partial Total Variation regularization (TV). The TV regularization is already used in cases the solution is expected to be piece-wise constant. By multiplying by $P(x_i)$, the reconstruction is driven towards the expected solution space while not being too restrictive in case the prior information is misleading.

Multiple configurations of measurement sensors number were tested to assess the reconstruction capacity with regard to the number of independent measurements. The solutions and execution speeds are compared to a classical FEM adjoint algorithm implemented with the dolfin-adjoint library.

## D. Uncertainty quantification

An ensemble of five conductivity configurations were randomly obtained. The inverse problems were computed by an ensemble of five neural networks. The final prediction considered was the mean of the five networks and the uncertainty their variance.

**E. Active Learning scheme**

It is now described an experiment of sensor placement scheme based on uncertainty estimates. With an initial set of $N_{M_i}$ electrodes uniformly placed around the volume of interest, a first estimation by the neural network is performed for both conductivity and potentials. The next measurement location should be at the point that reduces the potential estimation error the most. By having a better estimation of $\phi$, it is supposed that computing $\sigma$ through 14 should yield better overall results. This estimation error being unavailable in practice, it was decided to place the next measurement location at the point where the uncertainty is the highest. Since the measurements can only be performed on the boundaries, the inner variance among the different estimations made by the multiple networks is projected onto the surface $\partial\Omega$. To do so, it is computed the center of gravity and the method chooses the nearest $\partial\Omega$ point.

The model is trained while acquiring new data. The first estimation is performed through a full 30 000 epoch training, and each new measurement location allows the networks to be trained for another 10 000 epochs on those new data. A new estimate is available after each step of new measurements and training. This new estimate should be more accurate, is accompanied by a new uncertainty map. The process is repeated until one reaches an acceptable conductivity reconstruction or one runs out of sensors.

An ensemble of five conductivity configurations are randomly drawn. For each configuration, an initial number of $N_{M_i}$ = 8 measurement points were used to solve the inverse problem as described in the previous section and the quality of the reconstruction assessed by MAE. Then, an additional $N_{M_a}$ = 16 measurement points were added one by one to fine-tune the network. The Active Learning method is compared to a random measurement location scheme.

**Results**

**A. Analytical comparison**

The numerical results of the MAE are shown in Table I below:

Table I.

|  | MAE | Surface MAE | Max MAE |
|---|---|---|---|
| FEM | 0.0376 ( ±0.0195) | 0.0410 (±0.0225) | 0.0862(±0.0353) |
| Neural Network | 0.0301 (±0.0173) | 0.0389 (±0.0294) | 0.1149 (±0.05) |

From Table I, it can be observed that even though the PINN tends to show absolute error values whose maximum are higher than the FEM, these high values are outliers and the general reconstruction better fits the analytical solution than the FEM.

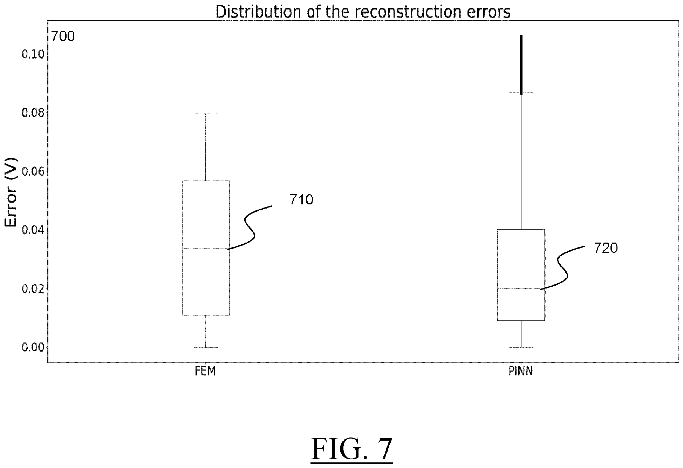

Reference is made to FIG. 7, illustrating a boxplot of the reconstruction error 700. The boxplot 710 shows the error concentration values 710 for the FEM, and the error concentration values 720 of the neural network (denoted as PINN). The main problem lies in the fact that those higher errors are concentrated near the surfaces and could affect the inverse problem reconstruction, which is based on surface measurements. By focusing solely on the surface, one still finds that the neural network outperforms the FEM method by a margin, despite showing the highest errors.

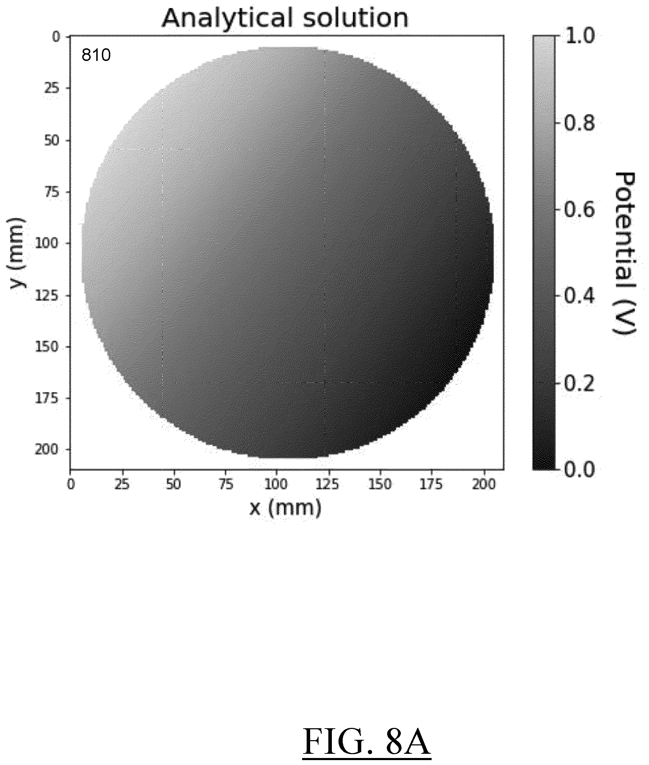

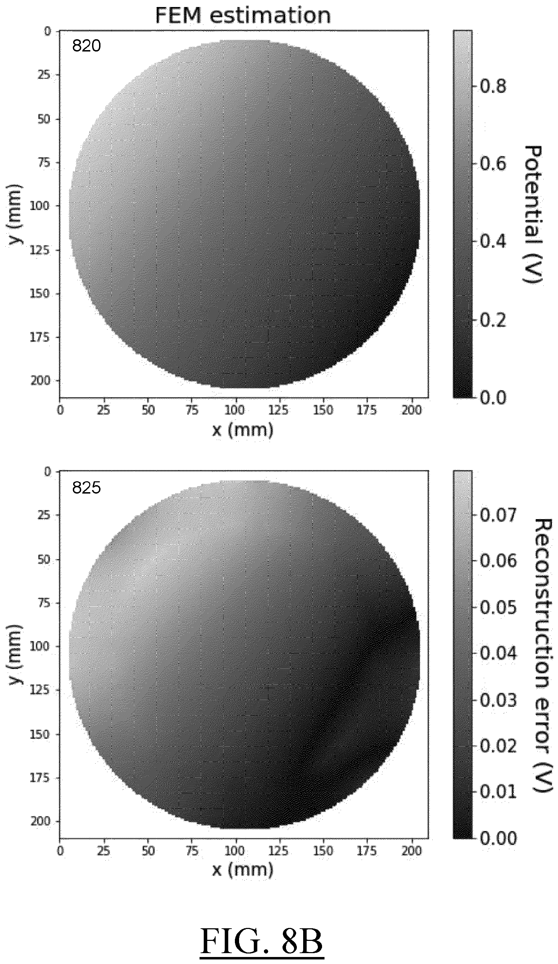

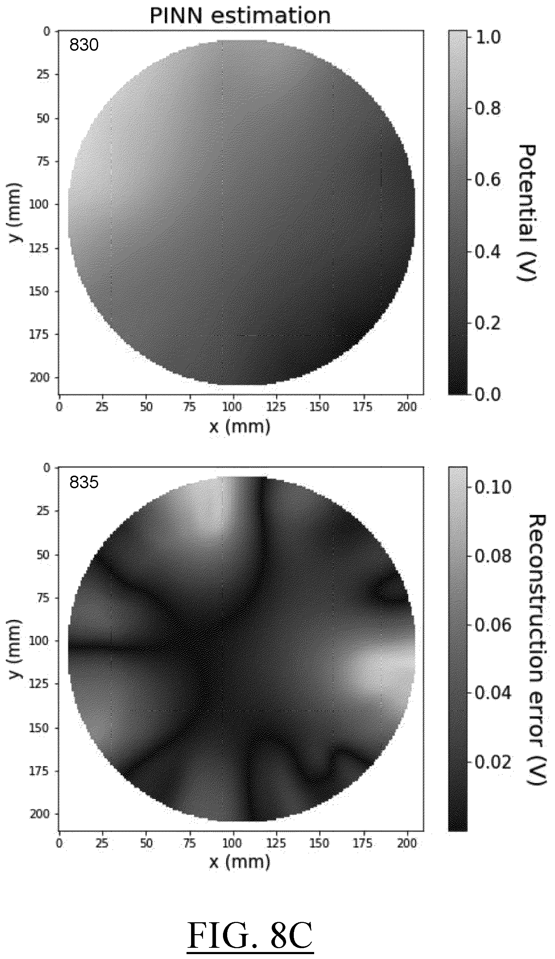

Reference is made to FIG.s 8A to 8C. FIG. 8A shows the solution of the direct problem using the analytical method. The plot 810 shows the analytical solution in the form of potential values (in Volts) over x and y coordinates (a higher level of gray means a higher potential). FIG. 8B shows the solution of the inverse problem using the state of the art FEM method. The plot 820 shows the estimated potential solved with the FEM method. The plot 825 illustrates the reconstruction error of the estimated potential obtained with the FEM method against the analytical solution. FIG. 8C shows the solution of the inverse problem by the neural network. The plot 830 shows the estimated potential solved with the neural network. The plot 835 illustrates the reconstruction error of the estimated potential obtained with the neural network against the analytical solution.

**B. Direct problem simulations**

The numerical results of the coil potentials are shown in Table II below.

Table II.

|  | X coil | Y coil | Z coil |
|---|---|---|---|
| MAE | 0.01295 (±0.00872) | 0.01254 (±0.00851) | 0.01618 (±0.00918) |

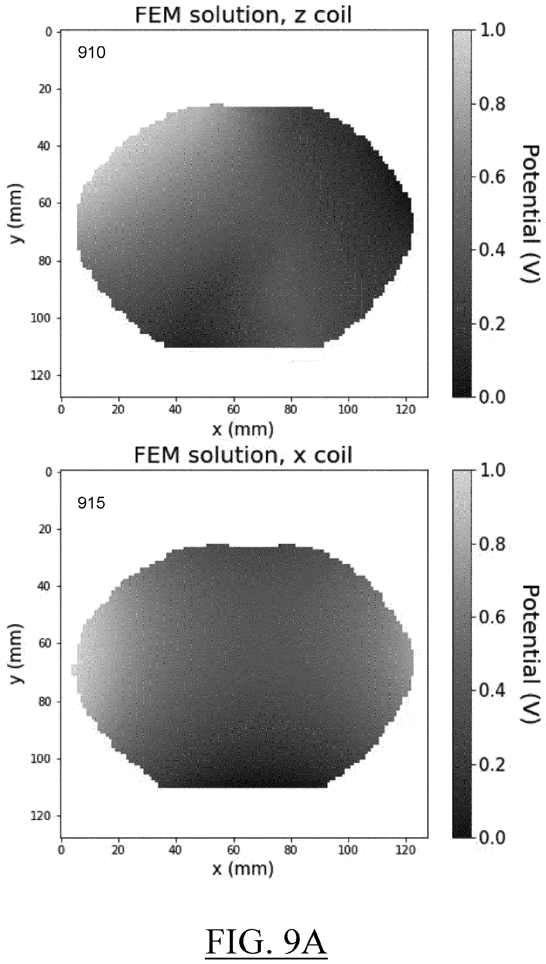

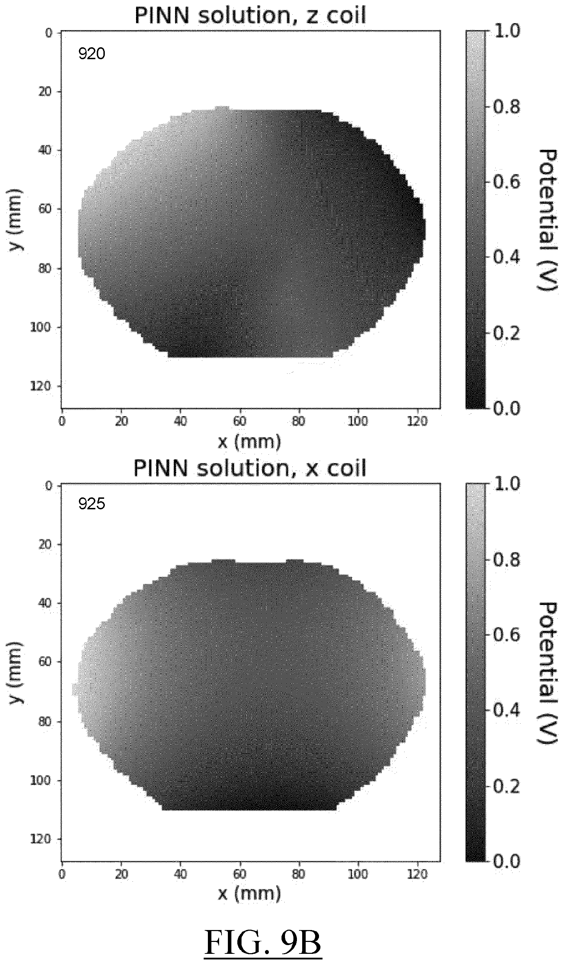

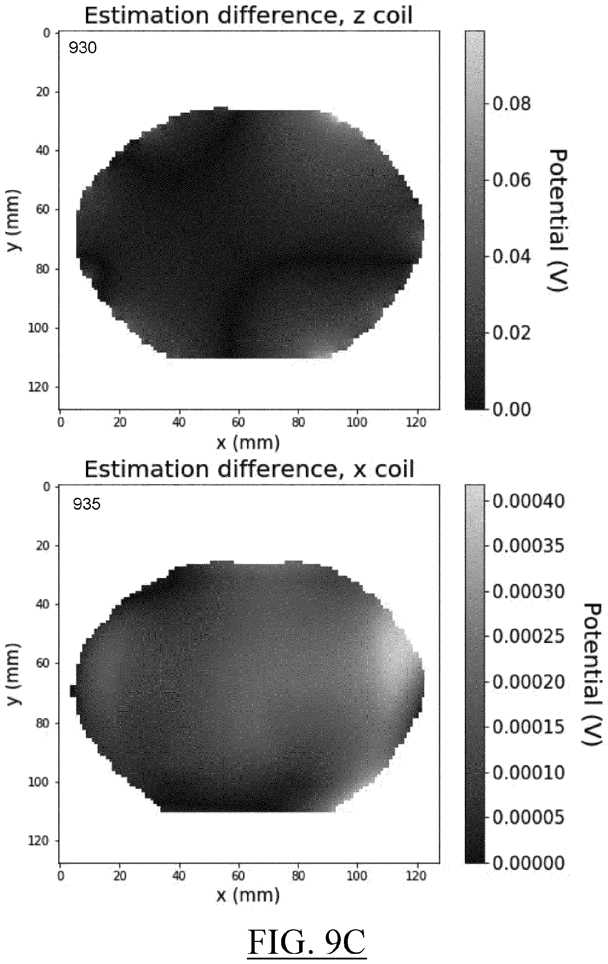

Reference is made to FIG.s 9A to 9C, showing the coil potentials. FIG. 9A shows the coil potentials estimated with the FEM. The plot 910 illustrates the estimation for the z coil, and the plot 915 shows the estimation with the x coil. FIG. 9B shows the coil potentials estimated with the neural network. The plot 920 illustrates the estimation for the z coil, and the plot 925 shows the estimation with the x coil. FIG. 9C shows the absolute reconstruction errors for the coil potentials. The plot 930 illustrates the absolute reconstruction error for the z coil, and the plot 935 shows the absolute reconstruction error for the x coil.

### C. Inverse problem

The comparison of the time needed for the estimation of $\phi$ are now shown in Table III below. The units of time are given in hours.

Comparison between FEM and PINN - 8 electrodes

Table III

| | PINN | | | FEM | | |
|---|---|---|---|---|---|---|
| Noise level (SNR) | Inf | 30dB | 20dB | Inf | 30dB | 20dB |
| MSE | 0.0286 | 0.0299 | 0.4531 | 0.0297 | 0.0758 | |
| Time | h | | | h | | |
| Comparison between FEM and PINN - 16 electrodes | | | | | | |
| | PINN | | | FEM | | |
| Noise level (SNR) | Inf | 30dB | 20dB | Inf | 30dB | 20dB |
| MSE | 0.0177 | 0.0260 | 0.1207 | 0.0257 | 0.0495 | |
| Time | h | | | h | | |
| Comparison between FEM and PINN - 32 electrodes | | | | | | |
| | PINN | | | FEM | | |
| Noise level (SNR) | Inf | 30dB | 20dB | Inf | 30dB | 20dB |
| MSE | 0.0074 | 0.0135 | 0.0286 | 0.0236 | 0.0280 | |
| Time | h | | | h | | |

### D. Uncertainty quantification

Fig.s 8A to 8C show that regions where the uncertainty estimate is the highest tend to correspond to the regions more prone to errors regarding the direct problem estimation. One thing that can be noticed is that the errors and uncertainties lie near the boundaries, hence providing an easy target for the next sensor's location.

Fig.s 9A to 9C show a situation where sensors are misplaced and a large zone is left without any measurement. As expected, the uncertainty and error are concentrated in the area deprived of sensors. Naturally, the barycenter of uncertainty/error, once projected on $\Omega$, is close to the one estimated by the networks.

Thus, the assumption that taking a new measurement in the region where the variance is the highest should reduce the error of the Neural Network seems in par with the results of sections A to C.

### E. Active Learning scheme

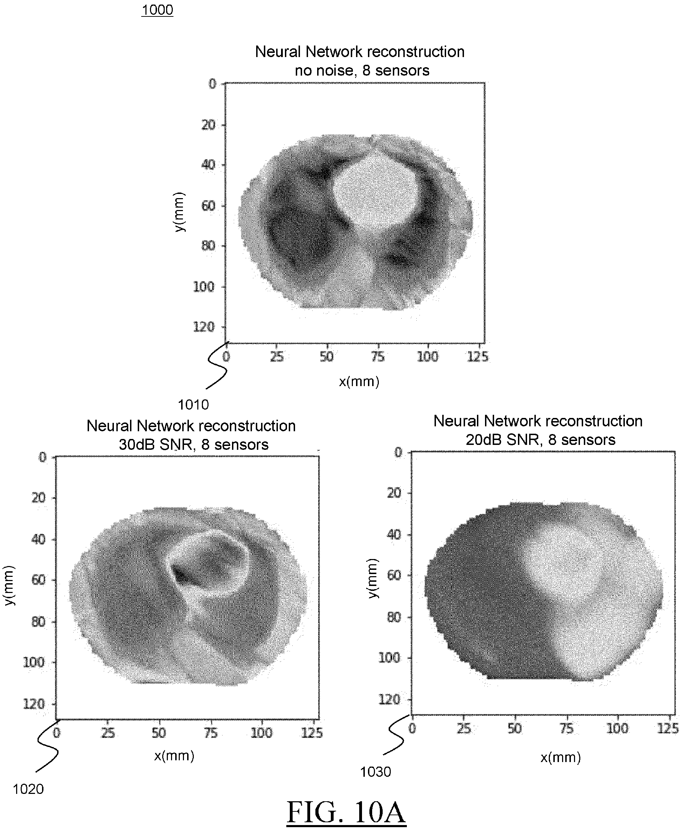

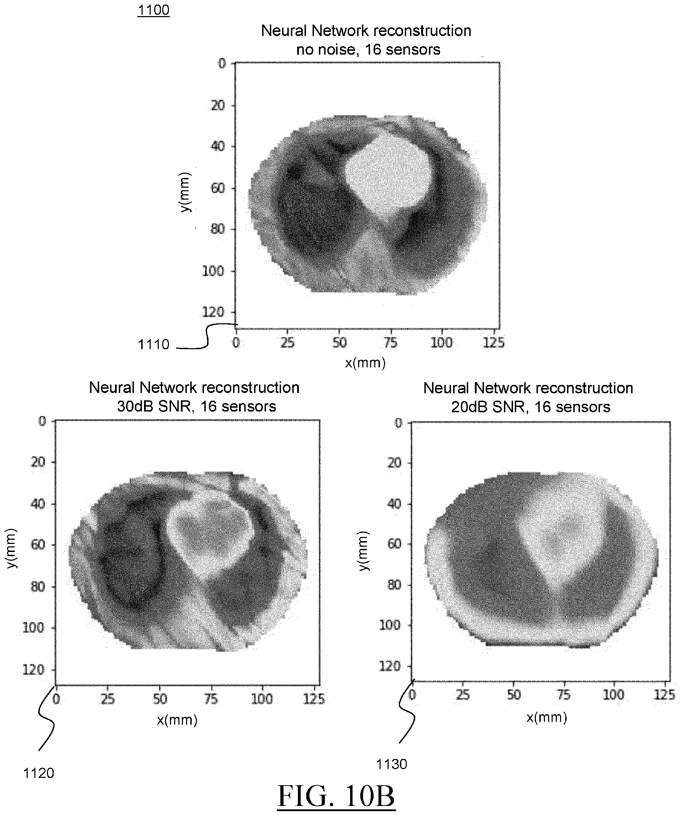

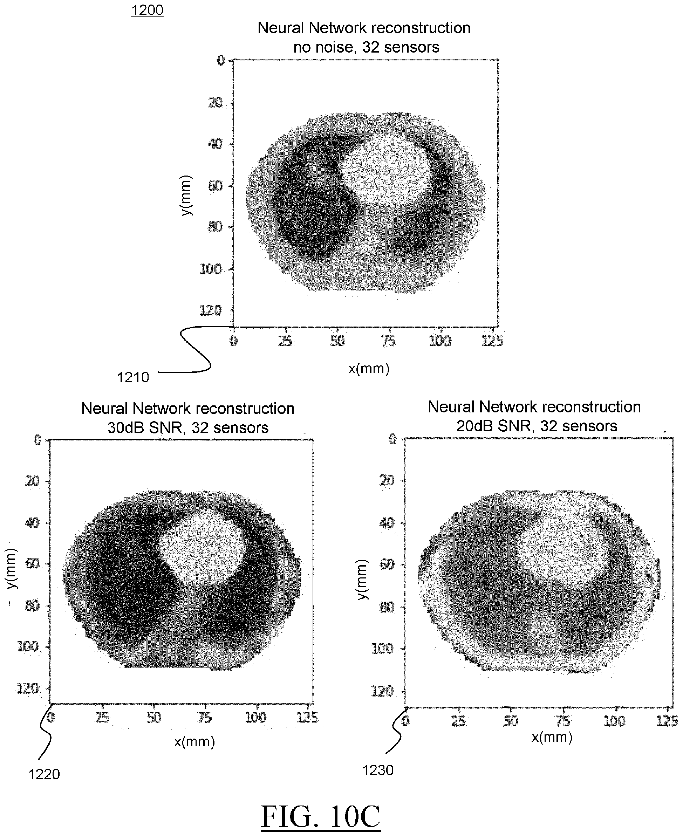

Fig.s 10A to 10C shows their improvements in the inverse problem's reconstruction according to the number of extra measurements. FIG. 10A shows the reconstruction with 8 placed sensors. The plot 1010 illustrates the quality of the reconstruction with no noise. The plot 1020 illustrates the quality of the reconstruction with a 30dB signal-to-noise (SNR) ratio. The plot 1030 illustrates the quality of the reconstruction with a 20dB SNR ratio. FIG. 10B shows the reconstruction with 16 placed sensors. The plot 1110 illustrates the quality of the reconstruction with no noise. The plot 1120 illustrates the quality of the reconstruction with a 30dB SNR ratio. The plot 1130 illustrates the quality of the reconstruction with a 20dB SNR ratio. FIG. 10C shows the reconstruction with 32 placed sensors. The plot 1210 illustrates the quality of the reconstruction with no noise. The plot 1220 illustrates the quality of the reconstruction with a 30dB SNR ratio. The plot 1230 illustrates the quality of the reconstruction with a 20dB SNR ratio.

**Claims**

1. A computer-implemented method for estimating a distribution of conductivity over a portion of a tissue of a body, the method comprising:

   • obtaining a magnetic resonance image (MRI) of the portion of tissue and a body surface electrical potential measurement associated with the MRI, the body surface electrical potential measurement stemming from measurements performed by two or more surface electrodes placed on the body during the MRI process; and
   • estimating the distribution of conductivity on the portion of tissue based on the body surface electrical potential measurement and on the MRI;

   wherein the estimation of the distribution of conductivity comprises obtaining a solution of an inverse problem over the portion of tissue, the solution of the inverse problem consisting of a distribution of one or more conductivity values satisfying a system of equations derived from Maxwell's equations associated with the body surface electrical potential measurement;
   wherein obtaining the solution of the inverse problem comprises:

   • learning a neural network based on the obtained MRI and the obtained body surface electrical potential measurement, the neural network being configured for estimating the distribution of conductivity on the portion of tissue as an approximated solution of the system of equations; and
   • using the learnt neural network to obtain the solution of the inverse problem, wherein using the learnt neural network comprises feeding the obtained MRI and the obtained body surface electrical potential measurement as input to the learnt neural network and outputting the estimated distribution of conductivity;

   wherein the learning comprises minimizing a loss, the loss comprising a term depending increasingly on residual functions corresponding to the system of equations, the residual functions quantifying an extent to which the system of equations is satisfied by an estimated distribution of conductivity on the portion of tissue; and
   wherein the loss is of the type:

   $$\mathcal{L}\left(\boldsymbol{\theta}_{\sigma}, \boldsymbol{\theta}_{\phi}\right) = \mathcal{L}_1(X_1) + \lambda_2\mathcal{L}_2(X_2) + \lambda_M\mathcal{L}_M(X_M) + \lambda_R\mathcal{L}_R(\boldsymbol{\theta}_{\sigma}) + \lambda_p\mathcal{L}_P(X_P);$$

   wherein:

   • $\mathcal{L}_1(X_1) = \frac{1}{N_1}\sum_{x_i \in X_1} R_1(\boldsymbol{x_i})^2$, with $R_1(x) := \nabla \cdot (\sigma(x)\nabla 0(x)) + j\omega \nabla \cdot (\sigma(x)A_P(x))$,

   • $\lambda_2\mathcal{L}_2(X_2) = \frac{\lambda_2}{N_2}\sum_{x_i \in X_2} R_2(\boldsymbol{x_i})^2$, with $R_2(x) := \frac{\partial \phi}{\partial n}(x) + j\omega A_P(x) \cdot \boldsymbol{n}$,

   • $\lambda_M\mathcal{L}_M(X_M) = \frac{\lambda_M}{N_M}\sum_{x_i \in X_M}(\phi(\boldsymbol{x_i}) - \Phi_i)^2$,

   • $\lambda_R\mathcal{L}_R(\Theta_{\sigma}) = \lambda_R \Sigma_{\Theta_i \in \Theta_{\phi}} \Theta_i^2$, and

   • $\lambda_p\mathcal{L}_P(X_P) = \frac{\lambda_p}{N_P}\sum_{x_i \in X_P}\|\nabla\sigma(\boldsymbol{x})\| \cdot P(\boldsymbol{x_i})$, with $P(\boldsymbol{x_i}) = \begin{cases} 1 \text{ if } \|\nabla S(\boldsymbol{x_i})\| = 0 \\ 0 \quad \text{otherwise} \end{cases}$, and where $S(\boldsymbol{x_i})$ is an element of prior tissue segmentation data S,

   and where:

   • **x** is a real-valued vector belonging to a domain $\Omega$ derived from the MRI,
   • $\sigma(\boldsymbol{x})$ represents the distribution of conductivity,

- $\phi(x)$ represents the electrical potential ,
- $R_1(x)$ and $R_2(x)$ represent the residual functions,

- $\mathcal{L}_M(X_M)$ is the term that penalizes non-respect of the body surface electrical potential measurement by an estimated electrical potential of the tissue,

- $\mathcal{L}_R(\Theta_\sigma)$ is an L2-regularization term of the body surface electrical potential measurement,

- $\mathcal{L}_P(X_P)$ is a term that penalizes a non-respect of prior tissue segmentation data S by the gradient of the estimated distribution of conductivity $\nabla\sigma(x)$,
- $\lambda_2$, $\lambda_M$, $\lambda_R$ and $\lambda_p$ are adjustable non-negative coefficients,
- $\theta_\sigma$ and $\theta_\phi$ represent parameters of the neural network,
- $X_1$ is a set of $N_1$ points of the domain $\Omega$,
- $X_2$ is a set of $N_2$ points of a boundary of the domain $\Omega$,
- $X_M$ is a set of $N_M$ points of the boundary of the domain $\Omega$ where the values $\Phi_i$ of $\phi(x_i)$, $x_i \in X_M$, are form the obtained body surface electrical potential measurement,
- $X_P$ is a set of $N_P$ points,

$j, \omega)$, $n$ and $\boldsymbol{A_P}$ are respectively the current density, angular frequency, the normal vector and the primary potential vector.

2. The method of claim 1, wherein the neural network is further configured for estimating an electrical potential of the tissue, the loss further comprising a term that penalizes non-respect of the body surface electrical potential measurement by an estimated electrical potential of the tissue.

3. The method of claim 1 or 2, wherein the loss further comprises a term that penalizes a non-respect of prior tissue segmentation data by a gradient of the estimated distribution of conductivity.

4. The method of any one of claims 1 to 3, wherein the neural network comprises a first sub-network and a second sub-network, the first sub-network comprising first parameters to be set during the learning, and the second sub-network comprising second parameters to remain fixed during the learning.

5. The method of claim 4, wherein the learning of the neural network is iterated, each iteration comprising modifying the second parameters of the second sub-network.

6. The method of claim 5, wherein the method further comprises computing a mean of the estimated distributions of conductivity obtained from each iteration of the learning and computing a variance of the distributions corresponding to the uncertainty of the distributions.

7. The method of claim 6, wherein the method further comprises performing a modified body surface electrical potential measurement based in the uncertainty, and re-learning the neural network based on the modified measurement.

8. A computer program comprising instructions which, when the program is executed by a computer, cause the computer to carry out the method of any of claims 1-7.

9. A computer-readable storage medium having recorded thereon the computer program of claim 8.

10. A system comprising a processor coupled to a memory, the memory having recorded thereon the computer program of claim 8.

**Patentansprüche**

1. Computerimplementiertes Verfahren zum Schätzen einer Verteilung von Leitfähigkeit über einen Abschnitt eines Körpergewebes, das Verfahren umfassend:

   • Erlangen eines Magnetresonanzbilds (MRI) des Gewebeabschnitts und einer Messung des elektrischen Körperoberflächenpotentials, die mit dem MRI assoziiert ist, wobei die Messung des elektrischen Körperoberflächenpotentials aus Messungen stammt, die von zwei oder mehreren Oberflächenelektroden durchgeführt werden, die während des MRI-Vorgangs auf dem Körper platziert werden; und
• Schätzen der Verteilung der Leitfähigkeit auf dem Gewebeabschnitt basierend auf der Messung des elektrischen Körperoberflächenpotentials und auf dem MRI;

wobei die Schätzung der Verteilung von Leitfähigkeit ein Erlangen einer Lösung eines inversen Problems über den Gewebeabschnitt umfasst, wobei die Lösung des inversen Problems aus einer Verteilung von einem oder mehreren Leitfähigkeitswerten besteht, die ein Gleichungssystem erfüllen, das aus Maxwell-Gleichungen abgeleitet ist, die mit der Messung des elektrischen Körperoberflächenpotentials assoziiert sind;
wobei ein Erlangen der Lösung des inversen Problems Folgendes umfasst:

• Lernen eines neuronalen Netzes basierend auf dem erlangten MRI und der erlangten Messung des elektrischen Körperoberflächenpotentials, wobei das neuronale Netz konfiguriert ist, um die Verteilung von Leitfähigkeit auf dem Gewebeabschnitt als eine angenäherte Lösung des Gleichungssystems zu schätzen; und
• Verwenden des gelernten neuronalen Netzes, um die Lösung des inversen Problems zu erlangen, wobei ein Verwenden des gelernten neuronalen Netzes ein Einspeisen des erlangten MRI und der erlangten Messung des elektrischen Körperoberflächenpotentials als Eingang in das gelernte neuronale Netz und ein Ausgeben der geschätzten Verteilung von Leitfähigkeit umfasst;

wobei das Lernen ein Minimieren eines Verlusts umfasst, der Verlust umfassend einen Term, der zunehmend von Restfunktionen abhängt, die dem Gleichungssystem entsprechen, wobei die Restfunktionen ein Ausmaß quantifizieren, in dem das Gleichungssystem durch eine geschätzte Verteilung von Leitfähigkeit auf dem Gewebeabschnitt erfüllt ist; und
wobei der Verlust von folgender Art ist:

$$\mathcal{L}\left(\boldsymbol{\theta}_{\sigma}, \boldsymbol{\theta}_{\phi}\right) = \mathcal{L}_1(X_1) + \lambda_2\mathcal{L}_2(X_2) + \lambda_M\mathcal{L}_M(X_M) + \lambda_R\mathcal{L}_R(\boldsymbol{\theta}_{\sigma}) + \lambda_p\mathcal{L}_P(X_P);$$

wobei:

• $\mathcal{L}_1(X_1) = \frac{1}{N_1}\sum_{x_i \in X_1} R_1(\boldsymbol{x}_i)^2$, wobei $R_1(\boldsymbol{x}) := \nabla \cdot (\sigma(\boldsymbol{x})\nabla\phi>(\boldsymbol{x})) + j\omega\nabla\cdot (\sigma(\boldsymbol{x})\boldsymbol{A_P}(\boldsymbol{x}))$,

• $\lambda_2\mathcal{L}_2(X_2) = \frac{\lambda_2}{N_2}\sum_{x_i \in X_2} R_2(\boldsymbol{x}_i)^2$, wobei $R_2(\boldsymbol{x}) := \frac{\partial\phi}{\partial n}(\boldsymbol{x}) + j\omega\boldsymbol{A_P}(\boldsymbol{x}) \cdot \boldsymbol{n}$,

• $\lambda_M\mathcal{L}_M(X_M) = \frac{\lambda_M}{N_M}\sum_{x_i \in X_M}(\phi(\boldsymbol{x}_i) - \Phi_i)^2$,

• $\lambda_R\mathcal{L}_R(\Theta_{\sigma}) = \lambda_R\Sigma_{\Theta_i \in \Theta_{\phi}}\Theta_i^2$, und

• $\lambda_p\mathcal{L}_P(X_P) = \frac{\lambda_p}{N_P}\sum_{x_i \in X_P}\|\nabla\sigma(\boldsymbol{x})\| \cdot P(\boldsymbol{x}_i)$, wobei

• $\lambda_2\mathcal{L}_2(X_2) = \frac{\lambda_2}{N_2}\sum_{x_i \in X_2} R_2(\boldsymbol{x}_i)^2$, wobei $P(\boldsymbol{x}_i) = \begin{cases} 1 \text{ wenn } \|\nabla S(\boldsymbol{x}_i)\| = 0 \\ 0 \text{ sonst} \end{cases}$ und

wobei $S(xi)$ ein Element von vorherigen Gewebesegmentierungsdaten S ist, und wobei:

• x ein Realwert-Vektor ist, der zu einem Bereich $\Omega$ gehört, der von dem MRI abgeleitet ist,
• $\sigma(x)$ die Verteilung von Leitfähigkeit darstellt,
• $\phi(x)$ das elektrische Potential darstellt,
• $R_1(x)$ und $R_2(\boldsymbol{x})$ die Restfunktionen sind,
• $L_M(X_M)$ der Term ist, der eine Nichteinhaltung der Messung des elektrischen Körperoberflächenpotentials durch ein geschätztes elektrisches Potential des Gewebes bestraft,
• $L_R(\theta_{\sigma})$ ein L2-Regularisierungsterm der Messung des elektrischen Körperoberflächenpotentials ist,
• $L_p(X_p)$ ein Term ist, der eine Nichteinhaltung von vorherigen Gewebesegmentierungsdaten S durch den Gradienten der geschätzten Verteilung von Leitfähigkeit $\nabla_{\sigma}(x)$ bestraft,
• $\lambda_2$, $\lambda_M$, $\lambda_R$ und $\lambda_p$ einstellbare nicht negative Koeffizienten sind,

• $\theta_\sigma$ und $\theta_\phi$ Parameter des neuronalen Netzes sind,
• $X_1$ eine Menge von $N_1$ Punkten des Bereichs $\Omega$ ist,
• $X_2$ ein Satz von $N_2$ Punkten einer Begrenzung des Bereichs $\Omega$ ist,
• $X_M$ eine Menge von $N_M$ Punkten des Rands des Bereichs $\Omega$ ist, in dem die Werte $\Phi_i$ von $\phi(x_i)$, $x_i \in X_M$ aus der Messung des elektrischen Körperoberflächenpotentials sind,
• $X_P$ ein Satz von $N_P$ Punkten ist,

$j, \omega, n$ und $A_P$ jeweils die Stromdichte, die Winkelfrequenz, der Normalenvektor bzw. der Primärpotentialvektor sind.

2. Verfahren nach Anspruch 1, wobei das neuronale Netz ferner konfiguriert ist, um ein elektrisches Potential des Gewebes zu schätzen, der Verlust ferner umfassend einen Term, der eine Nichteinhaltung der Messung des elektrischen Körperoberflächenpotentials durch ein geschätztes elektrisches Potential des Gewebes bestraft.

3. Verfahren nach Anspruch 1 oder 2, wobei der Verlust ferner einen Term umfasst, der eine Nichteinhaltung von vorherigen Gewebesegmentierungsdaten durch einen Gradienten der geschätzten Verteilung von Leitfähigkeit bestraft.

4. Verfahren nach einem der Ansprüche 1 bis 3, wobei das neuronale Netz ein erstes Teilnetz und ein zweites Teilnetz umfasst, das erste Teilnetz umfassend erste Parameter, die während des Lernens einzustellen sind, und das zweite Teilnetz umfassend zweite Parameter, die während des Lernens fest bleiben sollen.

5. Verfahren nach Anspruch 4, wobei das Lernen des neuronalen Netzes iteriert wird, jede Iteration umfassend ein Modifizieren der zweiten Parameter des zweiten Teilnetzes.

6. Verfahren nach Anspruch 5, wobei das Verfahren ferner ein Berechnen eines Mittelwerts der geschätzten Verteilungen von Leitfähigkeit, die aus jeder Iteration des Lernens erlangt werden, und ein Berechnen einer Varianz der Verteilungen, die der Unsicherheit der Verteilungen entspricht.

7. Verfahren nach Anspruch 6, wobei das Verfahren ferner ein Durchführen einer modifizierten Messung des elektrischen Körperoberflächenpotentials basierend auf der Unsicherheit und ein erneutes Lernen des neuronalen Netzes basierend auf der modifizierten Messung umfasst.

8. Computerprogramm, umfassend Anweisungen, die, wenn das Programm von einem Computer ausgeführt wird, den Computer veranlassen, das Verfahren nach einem der Ansprüche 1-7 durchzuführen.

9. Computerlesbares Speichermedium, auf dem das Computerprogramm nach Anspruch 8 aufgezeichnet ist.

10. System, umfassend einen Prozessor, der mit einem Speicher gekoppelt ist, wobei auf dem Speicher das Computerprogramm nach Anspruch 8 aufgezeichnet ist.

**Revendications**

1. Procédé mis en oeuvre par ordinateur pour estimer une distribution de conductivité sur une partie d'un tissu d'un corps, le procédé comprenant le fait de :

• obtenir une image par résonance magnétique (MRI) de la partie de tissu et une mesure de potentiel électrique de surface corporelle associée à l'image MRI, la mesure de potentiel électrique de surface corporelle provenant de mesures mises en oeuvre par deux électrodes de surface ou plus placées sur le corps pendant le processus MRI ; et
• estimer la distribution de conductivité sur la partie de tissu sur la base de la mesure de potentiel électrique de surface corporelle et de l'image MRI ;

dans laquelle l'estimation de la distribution de conductivité comprend le fait d'obtenir une solution d'un problème inverse sur la partie de tissu, la solution du problème inverse consistant en une distribution d'une ou plusieurs valeurs de conductivité satisfaisant un système d'équations dérivées des équations de Maxwell associées à la mesure de potentiel électrique de surface corporelle ;

dans lequel l'obtention de la solution du problème inverse comprend le fait de :

- apprendre un réseau de neurones sur la base de l'image MRI obtenue et de la mesure de potentiel électrique de surface corporelle obtenue, le réseau de neurones étant configuré de manière à estimer la distribution de conductivité sur la partie de tissu en tant qu'une solution approximée du système d'équations ; et
- utiliser le réseau de neurones appris en vue d'obtenir la solution du problème inverse, dans laquelle l'utilisation du réseau de neurones appris comprend le fait d'introduire l'image MRI obtenue et la mesure de potentiel électrique de surface corporelle obtenue en tant qu'entrée dans le réseau de neurones appris, et de fournir en sortie la distribution de conductivité estimée ;

dans lequel l'apprentissage comprend le fait de minimiser une perte, la perte comprenant un terme dépendant toujours plus de fonctions résiduelles correspondant au système d'équations, les fonctions résiduelles quantifiant la mesure dans laquelle le système d'équations est satisfait par une distribution de conductivité estimée sur la partie de tissu ; et

dans laquelle la perte est du type :

$$\mathcal{L}(\boldsymbol{\theta}_\sigma, \boldsymbol{\theta}_\phi) = \mathcal{L}_1(X_1) + \lambda_2 \mathcal{L}_2(X_2) + \lambda_M \mathcal{L}_M(X_M) + \lambda_R \mathcal{L}_R(\boldsymbol{\theta}_\sigma) + \lambda_p \mathcal{L}_P(X_P) ,$$

dans laquelle : •

$$\mathcal{L}_1(X_1) = \frac{1}{N_1} \sum_{x_i \in X_1} R_1(\boldsymbol{x_i})^2 \quad \text{ave } R_1(x) := \nabla \cdot (\sigma(x) \nabla \phi(x)) + j\omega \nabla \cdot (\sigma(x) A_P(x)), \bullet$$

$$\lambda_2 \mathcal{L}_2(X_2) = \frac{\lambda_2}{N_2} \sum_{x_i \in X_2} R_2(\boldsymbol{x_i})^2 \quad \text{, avec} \quad R_2(\boldsymbol{x}) := \frac{\partial \phi}{\partial n}(\boldsymbol{x}) + j\omega \boldsymbol{A_P}(\boldsymbol{x}) \cdot \boldsymbol{n} ,$$

$$\bullet \quad \lambda_M \mathcal{L}_M(X_M) = \frac{\lambda_M}{N_M} \sum_{x_i \in X_M} (\phi(\boldsymbol{x_i}) - \Phi_i)^2 ,$$

- $\lambda_R \mathcal{L}_R(\theta_\sigma) = \lambda_R \sum_{\theta_i \in \theta_\phi} \theta_i^2$, et

$$\bullet \quad \lambda_p \mathcal{L}_P(X_P) = \frac{\lambda_p}{N_P} \sum_{x_i \in X_P} \|\nabla \sigma(\boldsymbol{x})\| \cdot P(\boldsymbol{x_i}) \quad \text{avec} \quad P(\boldsymbol{x_i}) = \left\{ \begin{array}{l} 1 \text{ si } \|\nabla S(x_i)\| = 0 \\ 0 \text{ autrement} \end{array} \right.$$

et où S(xi) est un élément de données de segmentation de tissu antérieures S,

et où :

- x est un vecteur à valeur réelle appartenant à un domaine $\Omega$ dérivé de l'image MRI,
- $\sigma(x)$ représente la distribution de conductivité,
- $\phi(x)$ représente le potentiel électrique,
- $R_1(x)$ et $R_2(\boldsymbol{x})$ représentent les fonctions résiduelles,
- $\mathcal{L}_M(X_M)$ est le terme qui pénalise le non-respect de la mesure de potentiel électrique de surface corporelle par un potentiel électrique estimé du tissu,
- $\mathcal{L}_R(\theta_\sigma)$ est un terme de régularisation L2 de la mesure de potentiel électrique de surface corporelle,
- $\mathcal{L}_P(X_P)$ est un terme qui pénalise le non-respect de données de segmentation de tissu antérieures S par le gradient de la distribution de conductivité estimée $\nabla\sigma(x)$,
- $\lambda_2, \lambda_M, \lambda_R$ et $\lambda_p$ sont des coefficients non négatifs réglables,
- $\theta_\sigma$ et $\theta_\phi$ représentent des paramètres du réseau de neurones,
- $X_1$ est un ensemble de $N_1$ points du domaine $\Omega$,
- $X_2$ est un ensemble de $N_2$ points d'une frontière du domaine $\Omega$,
- $X_M$ est un ensemble de $N_M$ points de la frontière du domaine $\Omega$ où les valeurs $\Phi_i$ de $\phi(x_i)$, $x_i \in X_M$, proviennent de la mesure de potentiel électrique de surface corporelle obtenue,
- $X_P$ est un ensemble de $N_P$ points,

$j$, $\omega$, $n$ et $A_P$ sont respectivement la densité de courant, la fréquence angulaire, le vecteur normal et le vecteur de potentiel primaire.

2. Procédé selon la revendication 1, dans lequel le réseau de neurones est en outre configuré de manière à estimer un potentiel électrique du tissu, la perte comprenant en outre un terme qui pénalise le non-respect de la mesure de potentiel électrique de surface corporelle par un potentiel électrique estimé du tissu.

3. Procédé selon la revendication 1 ou 2, dans lequel la perte comprend en outre un terme qui pénalise le non-respect des données de segmentation de tissu antérieures par un gradient de la distribution de conductivité estimée.

4. Procédé selon l'une quelconque des revendications 1 à 3, dans lequel le réseau de neurones comprend un premier sous-réseau et un deuxième sous-réseau, le premier sous-réseau comprenant des premiers paramètres devant être définis pendant l'apprentissage, et le deuxième sous-réseau comprenant des deuxièmes paramètres destinés à rester définis pendant l'apprentissage.

5. Procédé selon la revendication 4, dans lequel l'apprentissage du réseau de neurones est itéré, chaque itération comprenant la modification des deuxièmes paramètres du deuxième sous-réseau.

6. Procédé selon la revendication 5, dans lequel le procédé comprend en outre le fait de calculer une moyenne des distributions de conductivité estimées obtenues à partir de chaque itération de l'apprentissage, et de calculer une variance des distributions correspondant à l'incertitude des distributions.

7. Procédé selon la revendication 6, dans lequel le procédé comprend en outre le fait de mettre en oeuvre une mesure de potentiel électrique de surface corporelle modifiée sur la base de l'incertitude, et de réapprendre le réseau de neurones sur la base de la mesure modifiée.

8. Programme informatique comprenant des instructions qui, lorsque le programme est exécuté par un ordinateur, amènent l'ordinateur à mettre en oeuvre le procédé selon l'une quelconque des revendications 1 à 7.

9. Support de stockage lisible par ordinateur sur lequel est enregistré le programme informatique selon la revendication 8.

10. Système comprenant un processeur couplé à une mémoire, sur laquelle mémoire est enregistré le programme informatique selon la revendication 8.

| obtaining a magnetic resonance image (MRI) of the portion of tissue and a body surface electrical potential measurement associated with the MRI | S10 |

| estimating the distribution of conductivity on the portion of tissue based on the body surface electrical potential measurement and on the MRI | S20 |

## FIG. 1

FIG. 2

$$\|\nabla\sigma\|_{\Omega} \cdot P$$

$$\|\phi - \phi_M\|_{\partial\Omega}$$

$$\|\nabla(\sigma\nabla\phi) - j\omega\nabla \cdot (A\sigma)\|_{\Omega}$$

$$\|\nabla\phi \cdot \vec{n} - \vec{A} \cdot \vec{n}\|_{\partial\Omega}$$

FIG. 3

400

410

R2

σ2 σ1

R1

420

R3

Coil

430

FIG. 4

FIG. 5A

530

## FIG. 5B

540

## FIG. 5C

FIG. 6

Distribution of the reconstruction errors

FIG. 7

FIG. 8A

FIG. 8B

FIG. 8C

FIG. 9A

FIG. 9B

FIG. 9C

1000

Neural Network reconstruction
no noise, 8 sensors

1010

Neural Network reconstruction
30dB SNR, 8 sensors

1020

Neural Network reconstruction
20dB SNR, 8 sensors

1030

## FIG. 10A

1100

Neural Network reconstruction
no noise, 16 sensors

1110

Neural Network reconstruction
30dB SNR, 16 sensors

Neural Network reconstruction
20dB SNR, 16 sensors

1120

1130

FIG. 10B

1200

Neural Network reconstruction
no noise, 32 sensors

1210

Neural Network reconstruction
30dB SNR, 32 sensors

1220

Neural Network reconstruction
20dB SNR, 32 sensors

1230

FIG. 10C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2018310854 A1
- WO 2007017779 A2
- US 8948875 B2

### Non-patent literature cited in the description

- Reconstruction of the Conductivity Profile of the Human Head by a Non-Invasive Method. **IBRAGIMOV Z.** 2019 International Conference on Electromagnetics in Advanced Applications. IEEE, 09 September 2019
- **KISTENEV YU. V.** Investigation of the electric field distribution in the human brain based on MRI and EEG data. *Proceedings of SPIE,* 16 April 2018, vol. 10614
- **YUQING WAN.** A feasibility study of magnetic resonance driven electrical impedance tomography using a phantom. *Physiological Measurement,* 29 May 2013, vol. 34 (6
- **LIU JIAEN.** Electrical Properties Tomography Based on B1 Maps in MRI: Principles, Applications and Challenges. *IEEE Transactions on Biomedical Engineering,* 21 August 2017, vol. 64 (11